# EUROPÄISCHE PATENTSCHRIFT

(11) **EP 4 193 143 B1**
(45) Veröffentlichungstag und Bekanntmachung des Hinweises auf die Patenterteilung: **28.01.2026**
(21) Anmeldenummer: 21754938.5
(22) Anmeldetag: 26.07.2021
(51) Int. Cl.: G01N 33/487, G16H 10/40, G01N 33/543, G16H 40/63

(54) **TESTVORRICHTUNG, TESTVORRICHTUNGSSYSTEM UND VERFAHREN ZUM FESTSTELLEN, OB EINE FLÜSSIGKEIT EINEN INHALTSSTOFF AUFWEIST**
TEST DEVICE, TEST DEVICE SYSTEM AND METHOD FOR DETERMINING WHETHER A LIQUID HAS A CONSTITUENT
DISPOSITIF DE TEST, SYSTÈME DE DISPOSITIF DE TEST ET PROCÉDÉ POUR DÉTERMINER SI UN LIQUIDE CONTIENT UN COMPOSANT

(30) Priorität: 06.08.2020 DE 102020210007
(43) Veröffentlichungstag der Anmeldung: 14.06.2023
(73) Patentinhaber: SCRIBOS GmbH, 69126 Heidelberg (DE)
(72) Erfinder: SCHEIBENSTOCK, Steffen, 70178 Stuttgart (DE); DÖRFEL, Fabian, 68259 Mannheim (DE); BORGSMÜLLER, Stefan, 69126 Heidelberg (DE)
(74) Vertreter: Zinsinger, Norbert
(86) Internationale Anmeldenummer: PCT/EP2021/070837
(87) Internationale Veröffentlichungsnummer: WO 2022/028936

(56) Entgegenhaltungen:
- WO-A1-2018/057801
- US-A1- 2010 045 789
- US-A1- 2013 230 845
- US-A1- 2020 152 339
- GÖKÇE ONUR ET AL: "High-Content Optical Codes for Protecting Rapid Diagnostic Tests from Counterfeiting", ANALYTICAL CHEMISTRY, vol. 90, no. 12, 19 June 2018 (2018-06-19), US, pages 7383 - 7390, XP055853599, ISSN: 0003-2700, DOI: 10.1021/acs.analchem.8b00826
- MTHEMBU CHRISTIAN L ET AL: "Google Analytics and quick response for advancement of gold nanoparticle-based dual lateral flow immunoassay for malaria - Plasmodium lactate dehydrogenase (pLDH)", 26 October 2017 (2017-10-26), pages 5943 - 5951, XP055853591, Retrieved from the Internet <URL:https://pubs.rsc.org/en/content/articlepdf/2017/ay/c7ay01645j> [retrieved on 20211021], DOI: 10.1039/c7ay01645j
- RUNGRAUNGSILP SUPPAT ET AL: "Data Hiding Method for QR Code Based on Watermark by compare DCT with DFT Domain", INTERNATIONAL CONFERENCE ON COMPUTER AND COMMUNICATION TECHNOLOGIES (ICCCT'2012), 2012, pages 144 - 148, XP055853557

## Beschreibung

Die vorliegende Erfindung betrifft eine Testvorrichtung und ein Verfahren zum Feststellen, ob eine Flüssigkeit einen Inhaltsstoff aufweist. Testvorrichtungen zum Feststellen, ob eine Flüssigkeit einen Inhaltsstoff aufweist sind aus dem Stand der Technik bekannt. Die aus dem Stand der Technik bekannten Testvorrichtungen weisen ein Probenfeld auf, auf das eine Probe einer Flüssigkeit, wie beispielsweise Blut, aufgebracht werden kann. Die Probe der Flüssigkeit kann einen Inhaltsstoff, wie beispielsweise Immunglobulin G oder Immunglobulin M, in mindestens einer Mindestmenge enthalten. Die aus dem Stand der Technik bekannten Testvorrichtungen weisen außerdem ein Ergebnisfeld auf, das ausgestaltet ist, eine erste Erscheinungsform anzunehmen, wenn die Probe den Inhaltsstoff in mindestens der Mindestmenge enthält, und eine zweite Erscheinungsform anzunehmen, wenn die Probe den Inhaltsstoff nicht in mindestens der Mindestmenge enthält. Anhand der ersten und zweiten Erscheinungsform kann auf das Vorhandensein des Inhaltsstoffs in mindestens der Mindestmenge oder auf das Vorhandensein des Inhaltsstoffs nicht in mindestens der Mindestmenge rückgeschlossen werden. Mit den aus dem Stand der Technik bekannten Testvorrichtungen kann also festgestellt werden, ob eine Flüssigkeit einen Inhaltsstoff aufweist.

Generell ist es wünschenswert, dass diese Testvorrichtungen dazu geeignet sind, dass eine bestimmte Testvorrichtung von anderen bestimmten Testvorrichtungen unterschieden werden kann, und gleichzeitig die Vervielfältigung der bestimmten Testvorrichtung verhindert oder zumindest wesentlich erschwert wird und ein Testergebnis als mit einer von einem bestimmten Hersteller hergestellten, nicht kopierten, Testvorrichtung ermittelt wurde.

Der Artikel GÖKÇE ONUR et al.: "High-Content Optical Codes for Protecting Rapid Diagnostic Tests from Counterfeiting", ANALYTICAL CHEMISTRY, Bd. 90, Nr. 12, 19. Juni 2018, Seiten 7383-7390, XP055853599, DOI: 10.1021/acs.analchem.8b00826 offenbart die Markierung von Teststreifen mit zweidimensionalen Mikrocodes, um deren Fälschung zu erschweren.

Es ist daher Aufgabe der vorliegenden Erfindung, die Sicherheit von Testergebnissen zu erhöhen. Gemäß einem ersten Aspekt der Erfindung wird die genannte Aufgabe durch eine Testvorrichtung mit den Merkmalen des Patentanspruchs 1 gelöst.

Die Testvorrichtung ist zum Feststellen, ob eine Flüssigkeit einen Inhaltsstoff aufweist, angepasst. Mithilfe der Testvorrichtung kann also festgestellt werden, ob die Flüssigkeit den Inhaltsstoff aufweist. Die Flüssigkeit kann beispielsweise Blut sein, sodass die Testvorrichtung ausgebildet ist, Blut auf das Vorhandensein eines bestimmten Inhaltsstoffs zu untersuchen. Alternativ kann die Flüssigkeit auch beispielsweise Urin sein, sodass die Testvorrichtung ausgebildet ist, Urin auf das Vorhandensein eines bestimmten Inhaltsstoffs zu untersuchen. Weiter kann die Flüssigkeit auch beispielsweise Speichel sein, sodass die Testvorrichtung ausgebildet ist, Speichel auf das Vorhandensein eines bestimmten Inhaltsstoffs zu untersuchen. Außerdem kann die Flüssigkeit auch beispielsweise eine andere Körperflüssigkeit, wie beispielsweise Schweiß, sein, sodass die Testvorrichtung ausgebildet ist, die Körperflüssigkeit, wie beispielsweise Schweiß, auf das Vorhandensein eines bestimmten Inhaltsstoffs zu untersuchen. Der Inhaltsstoff kann beispielsweise Immunglobulin G sein, sodass die Testvorrichtung ausgebildet ist, die Flüssigkeit auf das Vorhandensein von Immunglobulin G zu untersuchen. Alternativ kann der Inhaltsstoff auch beispielsweise Immunglobulin H sein, sodass die Testvorrichtung ausgebildet ist, die Flüssigkeit auf das Vorhandensein von Immunglobulin H zu untersuchen. Weiter kann der Inhaltsstoff auch beispielsweise Immunglobulin M sein, sodass die Testvorrichtung ausgebildet ist, die Flüssigkeit auf das Vorhandensein von Immunglobulin M zu untersuchen. Insbesondere kann der Inhaltsstoff ein Antikörper, wie beispielsweise Immunglobulin G, Immunglobulin H oder Immunglobulin M. Der Inhaltsstoff kann jedoch auch ein Antigen sein. Weiter kann die Testvorrichtung zum Feststellen angepasst sein, ob die Flüssigkeit einen ersten Inhaltsstoff und einen zweiten Inhaltsstoff aufweist. In diesem Fall kann der erste Inhaltsstoff Immunglobulin G und der zweite Inhaltsstoff Immunglobulin H sein, sodass die Testvorrichtung ausgebildet ist, die Flüssigkeit auf das Vorhandensein von Immunglobulin G und Immunglobulin H zu untersuchen. Weiter kann die Testvorrichtung zum Feststellen angepasst sein, ob die Flüssigkeit eine Vielzahl bestimmter Inhaltsstoffe aufweist. Die im Zusammenhang mit dem Inhaltsstoff und der Flüssigkeit beschriebenen Merkmale, technischen Effekte und/oder Vorteile gelten zumindest in analoger Weise auch für den ersten Inhaltsstoff, den zweiten Inhaltsstoff und die Vielzahl bestimmter Inhaltsstoffe, sodass an dieser Stelle auf eine entsprechende Wiederholung verzichtet wird. Beispielsweise können für jeden dieser Inhaltsstoffe sich unterscheidende Mindestmengen und erste und zweite Erscheinungsformen vorgesehen sein.

Die Testvorrichtung weist ein Probenfeld auf, auf das eine Probe der Flüssigkeit aufgebracht werden kann. Bevorzugt ist die Probe der Flüssigkeit eine kleine Menge der Flüssigkeit. Insbesondere kann die Flüssigkeit auch als zu testende Flüssigkeit bezeichnet werden. Weiter weist die Testvorrichtung ein Ergebnisfeld auf, das ausgestaltet ist, eine erste Erscheinungsform anzunehmen, wenn die Probe den Inhaltsstoff in mindestens einer Mindestmenge enthält. Wenn das Ergebnisfeld die erste Erscheinungsform annimmt kann darauf rückgeschlossen werden, dass die Probe den Inhaltsstoff in mindestens der Mindestmenge enthält.

Weiter ist das Ergebnisfeld ausgestaltet, eine zweite Erscheinungsform anzunehmen, wenn die Probe den Inhaltsstoff nicht in mindestens der Mindestmenge enthält. Wenn das Ergebnisfeld die zweite Erscheinungsform annimmt, kann darauf rückgeschlossen werden, dass die Probe den Inhaltsstoff nicht in mindestens der Mindestmenge enthält. Bevorzugt unterscheiden sich die erste Erscheinungsform und die zweite Erscheinungsform voneinander. Dies wird beispielsweise dadurch erreicht, dass die erste Erscheinungsform des Ergebnisfelds einen waagerechten Streifen in einem Kontrollfeld des Ergebnisfelds aufweist und die zweite Erscheinungsform des Ergebnisfelds den waagerechten Streifen in dem Kontrollfeld des Ergebnisfelds nicht aufweist. Alternativ kann dies beispielsweise dadurch erreicht werden, dass die erste Erscheinungsform des Ergebnisfelds den waagerechten Streifen in dem Kontrollfeld des Ergebnisfelds nicht aufweist und die zweite Erscheinungsform des Ergebnisfelds den waagerechten Streifen in dem Kontrollfeld des Ergebnisfelds aufweist. An dieser Stelle sei erwähnt, dass die vorliegende Erfindung nicht darauf beschränkt ist, dass die erste Erscheinungsform und die zweite Erscheinungsform sich durch das Erscheinen oder nicht Erscheinen eines waagerechten Streifen voneinander unterscheiden. Vielmehr können sich die erste Erscheinungsform und die zweite Erscheinungsform auch durch andere Merkmale, wie beispielsweise durch das Erscheinen eines waagerechten Streifens an unterschiedlichen Positionen oder durch das Erscheinen oder nicht Erscheinen von einer ausgefüllten Fläche oder nicht ausgefüllten Umrandungen, wie beispielsweise Kreisen, unterscheiden.

Außerdem weist die Testvorrichtung ein Sicherheitsfeld auf. Das Sicherheitsfeld kann unterschiedlich ausgebildet sein, wie dies im Folgenden näher ausgeführt ist. Dadurch, dass die Testvorrichtung sowohl das Ergebnisfeld als auch das Sicherheitsfeld aufweist, ist es möglich, das Ergebnisfeld und das Sicherheitsfeld einander zuzuordnen, beispielsweise nachdem das Ergebnisfeld und das Sicherheitsfeld gemeinsam von einer Erfassungseinheit optisch erfasst wurden, beispielsweise gescannt oder fotografiert wurden.

Das Sicherheitsfeld weist ein Informationsfeld mit einer Struktur auf. Insbesondere ist die Struktur eine zweidimensionale Darstellung, die so angeordnet ist, dass dann, wenn das Sicherheitsfeld optisch erfasst wird, die zweidimensionale Darstellung ebenfalls optisch erfasst wird. Die zweidimensionale Darstellung der Struktur weist ein Fourier-Muster auf oder ist aus diesem gebildet. Insbesondere ist vorgesehen, dass die Struktur selbst kein QR-Code, DataMatrix, Cool-Data-Matrix, Aztec, UPCode, Trillcode, Quickmark, Shotcode, mCode, oder Beetagg ist. Die Struktur kann vielmehr in bestimmten Ausführungsbeispielen einen Abschnitt eines QR-Codes, DataMatrix, Cool-Data-Matrix,

Aztecs, UPCodes, Trillcodes, Quickmarks, Shotcodes, mCodes, oder Beetaggs bilden. Die Struktur kann auch als Sicherheitsstruktur bezeichnet werden. Mit der Struktur ist ein erster eindeutiger Identifizierungscode kodiert. Durch Erfassen der Struktur und Dekodieren der erfassten Struktur kann somit der erste eindeutige Identifizierungscode ermittelt werden. Der erste eindeutige Identifizierungscode ist eindeutig und unterscheidet sich somit insbesondere von anderen eindeutigen Identifizierungscodes. Der eindeutige Identifizierungscode kann auch als unique identifier (UID) oder unique ID bezeichnet werden. Der erste eindeutige Identifizierungscode unterscheidet sich durch seine Eindeutigkeit insbesondere von anderen ersten eindeutigen Identifizierungscodes. Insbesondere ist vorgesehen, dass keine zwei Strukturen existieren, mit denen jeweils ein erster eindeutiger Identifizierungscode kodiert ist, wobei die beiden ersten eindeutigen Identifizierungscodes identisch sind. Dadurch, dass der erste eindeutige Identifizierungscode eindeutig ist, kann anhand des eindeutigen Identifizierungscodes auf die Identität der Testvorrichtung rückgeschlossen werden. Insbesondere kann dadurch, dass der erste eindeutige Identifizierungscode eindeutig ist, anhand des eindeutigen Identifizierungscodes die Testvorrichtung von anderen Testvorrichtungen, die gegebenenfalls identisch oder ähnlich aussehen, unterschieden werden. Der erste eindeutige Identifizierungscode ist mit der Struktur kodiert und liegt somit in kodierter Form vor, wobei die Struktur diese kodierte Form darstellt. Dadurch, dass der erste eindeutige Identifizierungscode kodiert ist, kann ein Benutzer der Testvorrichtung nicht zwangsläufig ohne technische Hilfsmittel, wie beispielsweise einer Erfassungseinheit und/oder Auswerteeinheit, auf den ersten eindeutigen Identifizierungscode rückschließen. Insbesondere ist es vorteilhaft, wenn die Struktur so ausgebildet ist, dass ein Benutzer der Testvorrichtung nicht ohne technische Hilfsmittel auf den ersten eindeutigen Identifizierungscode rückschließen kann. Wenn der Benutzer der Testvorrichtung nicht, insbesondere nicht zwangsläufig, ohne technische Hilfsmittel auf den ersten eindeutigen Identifizierungscode rückschließen kann, wird gewährleistet, dass eine Vervielfältigung der Testvorrichtung mit dem ersten eindeutigen Identifizierungscode wesentlich erschwert oder sogar verhindert wird.

Der erste eindeutige Identifizierungscode kann aus der Struktur nur dekodiert werden, wenn diese mit einer Mindestauflösung hergestellt ist. Das Herstellen der Struktur kann auch als Erzeugen der Struktur bezeichnet werden. Insbesondere ist die Struktur so fein, dass, insbesondere zu deren Herstellung, eine Mindestauflösung notwendig ist, damit die Struktur so dargestellt werden kann, dass der erste eindeutige Identifizierungscode aus der Struktur dekodiert werden kann. Die Struktur ist als Fourier-Muster ausgebildet.

Die Mindestauflösung, mit der die Struktur hergestellt ist, ist bevorzugt 300 dpi. Insbesondere hat es sich als vorteilhaft erwiesen, wenn die Mindestauflösung größer als 300 dpi ist. Noch vorteilhafter ist es, wenn die Mindestauflösung mit der die Struktur hergestellt ist, 600 dpi ist. Noch vorteilhafter ist es, wenn die Mindestauflösung, mit der die Struktur hergestellt ist, größer als 600 dpi ist. Noch vorteilhafter ist es, wenn die Mindestauflösung, mit der die Struktur hergestellt ist 800 dpi ist. Noch vorteilhafter ist es, wenn die Mindestauflösung, mit der die Struktur hergestellt ist, größer als 800 dpi ist. Mit zunehmender Mindestauflösung, mit der die Struktur hergestellt ist, erhöht sich der Schutz der Struktur vor einer Vervielfältigung der Struktur, da mit zunehmender Mindestauflösung insbesondere die Herstellung der Struktur erschwert wird, sodass diese nach der Herstellung so ausgebildet ist, dass der erste eindeutige Identifizierungscode aus der Struktur dekodiert werden kann. Weiter wird insbesondere bei einem Kopierversuch der Struktur, diese derart verändert oder besser gesagt bei dem Versuch der Reproduktion derart verfälscht, dass, insbesondere nach einer erneuten Herstellung, ein erneutes Dekodieren nicht mehr möglich ist. Insbesondere dass die Struktur als Fourier-Muster ausgebildet ist, gewährleistet eine hohe Mindestauflösung einen Kopierschutz, und zwar derart, dass die Struktur, nachdem sie optisch erfasst wurde, wie beispielsweise durch Scannen oder abfotografieren, und gegebenenfalls nach einem Reproduzieren der Struktur durch beispielsweise Ausdrucken der Struktur mit einem Standarddrucker, nicht mehr lesbar in dem Sinne ist, dass der erste eindeutige Identifizierungscode nicht aus der reproduzierten Struktur dekodiert werden kann. Dann kann darauf rückgeschlossen werden, dass die reproduzierte Struktur eine Kopie der Struktur ist, und somit ebenfalls darauf rückgeschlossen werden, dass beispielsweise eine Testvorrichtung mit angebrachter reproduzierter Struktur eine unerlaubte Kopie einer Testvorrichtung mit der Struktur ist, die für den Kopiervorgang optisch erfasst wurde. Zusammenfassend kann festgestellt werden, dass dadurch, dass der erste eindeutige Identifizierungscode aus der Struktur nur dann dekodiert werden kann, wenn diese mit einer Mindestauflösung hergestellt ist, ein Kopierschutz für die Testvorrichtung bereitgestellt werden kann. Insbesondere kann dadurch, dass der erste eindeutige Identifizierungscode aus der Struktur nur dann dekodiert werden kann, wenn diese mit einer Mindestauflösung hergestellt ist, gewährleistet werden, dass auf eine reproduzierte Struktur aus einer Original-Struktur rückgeschlossen werden kann.

Insbesondere können in einer bevorzugten Ausführungsform reflektierende Beschichtungen auf oder neben der Struktur vorgesehen sein, die ein optisches Erfassen der Struktur erschweren. In diesem Fall ist vorgesehen, dass beim gemeinsamen, vorzugsweise gleichzeitigen, optischen Erfassen des Ergebnisfelds und des Sicherheitsfelds die Struktur fokussiert ausgeleuchtet wird. Somit wird die Sicherheit weiter erhöht, da bei nicht fokussierter Ausleuchtung das Erfassen der Struktur erschwert wird.

Die Struktur oder das Informationsfeld mit der Struktur können auf die Testvorrichtung, insbesondere auf einen Grundkörper der Testvorrichtung, gedruckt werden, beispielsweise mittels digitalem Offsetdruck. Ebenso kann die Struktur oder das Informationsfeld mit der Struktur auf ein Etikett, insbesondere ein Grundkörper des Etiketts, gedruckt werden, wobei das Etikett eine selbstklebende Seite aufweist und mittels dieser selbstklebenden Seite stoffschlüssig mit einer Testvorrichtung verbunden werden kann. Insbesondere gewährleistet die Verbindung mit der Testvorrichtung ein Anbringen an der Testvorrichtung, sodass das Sicherheitsfeld des Etiketts das Sicherheitsfeld der Testvorrichtung bildet. Weiterhin kann die Struktur oder das Informationsfeld mit der Struktur auch in die Oberfläche der Testvorrichtung eingestanzt werden. Die Struktur oder das Informationsfeld mit der Struktur können auch mittels Laserbeschriftungsverfahren in die Oberfläche der Testvorrichtung eingeschrieben, bzw. eingebrannt, werden. Insbesondere das Einstanzen und das Laserbeschriften erzeugen untrennbare Verbindungen zwischen der Testvorrichtung und der Struktur oder der Testvorrichtung und dem Informationsfeld mit der Struktur, wodurch eine Manipulation, beispielsweise ein Austausch der Struktur, vermieden werden kann. Wenn im Zusammenhang mit der vorliegenden Erfindung von einem Anbringen des Etiketts an der Testvorrichtung, einem Verbinden des Etiketts mit der Testvorrichtung, von einem Drucken der Struktur oder des Informationsfelds mit der Struktur auf die Testvorrichtung, einem Einstanzen der Struktur oder des Informationsfelds mit der Struktur in die Oberfläche der Testvorrichtung, einem Einschreiben oder Einbrennen der Struktur oder des Informationsfelds mit der Struktur in die Oberfläche der Testvorrichtung beschrieben wird, so ist hiermit insbesondere gemeint, dass die entsprechenden Schritte durchgeführt werden und nach dem Durchführen der Schritte eine erfindungsgemäße Testvorrichtung hergestellt ist.

Weiter sind das Ergebnisfeld und das Sicherheitsfeld derart angeordnet, dass das Ergebnisfeld und das Sicherheitsfeld gemeinsam, vorzugsweise gleichzeitig, von einer Erfassungseinheit optisch erfasst werden können. Dadurch, dass das Ergebnisfeld und das Sicherheitsfeld derart angeordnet sind, dass das Ergebnisfeld und das Sicherheitsfeld gemeinsam, vorzugsweise gleichzeitig, von einer Erfassungseinheit optisch erfasst werden können, wird gewährleistet, dass das Ergebnisfeld und das Sicherheitsfeld gemeinsam, vorzugsweise gleichzeitig, insbesondere automatisch, von einer Erfassungseinheit optisch erfasst werden können. Weiter wird dadurch, dass das Ergebnisfeld und das Sicherheitsfeld derart angeordnet sind, dass das Ergebnisfeld und das Sicherheitsfeld gemeinsam, vorzugsweise gleichzeitig, von einer Erfassungseinheit optisch erfasst werden können, gewährleistet, dass auf ein Testergebnis anhand des optisch erfassten Ergebnisfelds, insbesondere anhand davon, ob das Ergebnisfeld die erste Erscheinungsform oder die zweite Erscheinungsform annimmt, und auf den ersten eindeutigen Identifizierungscode anhand des optisch erfassten Sicherheitsfelds rückgeschlossen werden kann. Beispielsweise kann so, insbesondere dadurch, dass der erste eindeutige Identifizierungscode kodiert in der Struktur vorliegt und die Struktur nur dekodiert werden kann, wenn diese mit einer Mindestauflösung hergestellt ist, auf eine zugelassene und von einem bestimmten Hersteller hergestellte Testvorrichtung sowie gleichzeitig auf ein bestimmtes Testergebnis rückgeschlossen werden. Weiter können die das Testergebnis und der erste eindeutige Identifizierungscode einander zugeordnet werden. Durch die Zuordnung des Testergebnisses und des ersten eindeutigen Identifizierungscodes kann diese Zuordnung in weiteren Auswertungen, wie beispielsweise in einem Vergleichen des ersten eindeutigen Identifizierungscodes mit gespeicherten eindeutigen Identifizierungscodes, verwendet werden. Weiter kann durch diese Zuordnung darauf rückgeschlossen werden, dass das bestimmte Testergebnis mit einer Testvorrichtung ermittelt wurde, die eine Struktur aufweist, die keine unerlaubt reproduzierte Struktur ist. Weiter kann durch das gemeinsame, vorzugsweise gleichzeitige optische Erfassen des Ergebnisfelds und des Sicherheitsfelds gewährleistet werden, dass eine Aufnahme eine Abbildung des Testergebnis und der Struktur aufweist und somit in einfacher Weise in einem Datensatz einander zugeordnet sein können, sodass eine Manipulation weiter erschwert wird. Insbesondere kann durch gleichzeitiges Erfassen, insbesondere durch Abfotografieren, eine Abbildung des Testergebnis und der Struktur in einer Aufnahme erfasst und somit miteinander in einem Datensatz "verbunden" sein, wodurch Manipulationen an dem abfotografierten Testergebnis erschwert werden.

Zusammenfassend kann also festgestellt werden, dass die vorliegende Erfindung die Sicherheit von Testergebnissen erhöht.

Erfindungsgemäß ist der erste eindeutige Identifizierungscode ein erster zweidimensionaler Code und die Struktur des Informationsfelds eine zweidimensionale Darstellung einer binarisierten Fourier-Transformierten einer den ersten zweidimensionalen Code repräsentierenden zweidimensionalen Funktion. Da die Struktur eine zweidimensionale Darstellung der binarisierten Fourier-Transformierten ist, kann die zweidimensionale Darstellung ein aus schwarzen und weißen Pixeln bestehendes Muster aufweisen oder aus diesem gebildet sein. Die Ausgestaltung ist aber nicht auf die Farben weiß und schwarz beschränkt. Insbesondere ist jede farbliche Darstellung geeignet, die in der Darstellung ausreichend Kontraste aufweist, um Pixel voneinander zu unterscheiden. Die zweidimensionale Darstellung der binarisierten Fourier-Transformierten kann auch als ein Fourier-Muster bezeichnet werden. Der erste zweidimensionale Code kann ein QR-Code, eine DataMatrix, eine Cool-Data-Matrix, ein Aztec, ein UPCode, ein Trillcode, Quickmark, Shotcode, mCode, Beetagg sein. Der erste zweidimensionale Code ist jedoch nicht auf einen Code aus dieser Liste beschränkt. Wie bereits beschrieben kann der erste eindeutige Identifizierungscode ein erster zweidimensionaler Code sein. Alternativ kann der erste eindeutige Identifizierungscode auch ein erster eindimensionaler Code sein. Der erste eindimensionale Code kann ein Code39, Code 128, Codebar, Interleaved 2of5, UPC, EAN, GS1, oder PDF417 sein. Der erste eindimensionale Code ist jedoch nicht auf einen Code aus dieser Liste beschränkt.

In einer Ausführungsform sind das Ergebnisfeld und das Sicherheitsfeld voneinander beabstandet. Dadurch, dass das Ergebnisfeld und das Sicherheitsfeld voneinander beabstandet sind, wird gewährleistet, dass zwischen dem Ergebnisfeld und dem Sicherheitsfeld weitere Informationen auf der Testvorrichtung abgebildet sein können.

In einer Ausführungsform grenzen das Ergebnisfeld und das Sicherheitsfeld aneinander an. Dadurch, dass das Ergebnisfeld und das Sicherheitsfeld aneinander angrenzen, wird gewährleistet, dass die optische Erfassung des Ergebnisfelds und des Sicherheitsfelds besonders einfach und platzsparend ermöglicht wird.

In einer Ausführungsform umschließt das Sicherheitsfeld das Ergebnisfeld. Wenn das Sicherheitsfeld das Ergebnisfeld umschließt, wird gewährleistet, dass bei der optischen Erfassung das Sicherheitsfeld als Orientierungshilfe verwendet werden kann und bei der optischen Erfassung auf die Anordnung des Ergebnisfelds anhand der Anordnung des Sicherheitsfeld rückgeschlossen werden kann. Zudem kann sichergestellt werden, dass bei einer optischen Erfassung des Sicherheitsfeldes, in jedem Fall das Ergebnisfeld miterfasst wird. Dadurch kann vermieden werden, dass ein Sicherheitsfeld beispielsweise abfotografiert wird, um dieses zu überprüfen, dabei versehentlich jedoch das Ergebnisfeld nicht miterfasst wird.

In einer Ausführungsform umschließt das Informationsfeld das Ergebnisfeld. Wenn das Informationsfeld das Ergebnisfeld umschließt, wird gewährleistet, dass bei der optischen Erfassung das Informationsfeld als Orientierungshilfe verwendet werden kann und bei der optischen Erfassung auf die Anordnung des Ergebnisfelds anhand der Anordnung des Informationsfelds rückgeschlossen werden kann. Zudem kann sichergestellt werden, dass bei einer optischen Erfassung des Informationsfelds, in jedem Fall das Ergebnisfeld miterfasst wird. Dadurch kann vermieden werden, dass ein Informationsfeld beispielsweise abfotografiert wird, um dieses zu überprüfen, dabei versehentlich jedoch das Ergebnisfeld nicht miterfasst wird.

Erfindungsgemäß weist das Sicherheitsfeld einen zweiten zweidimensionalen Code auf. Der zweite zweidimensionale Code kann ein QR-Code, eine DataMatrix, eine Cool-Data-Matrix, ein Aztec, ein UPCode, ein Trillcode, Quickmark, Shotcode, mCode, Beetagg sein. Der zweite zweidimensionale Code ist jedoch nicht auf einen Code aus dieser Liste beschränkt. Mit dem zweiten zweidimensionalen Code wird eine Möglichkeit bereitgestellt, weitere Informationen auf der Testvorrichtung anzuordnen. Zusätzlich kann das Sicherheitsfeld einen zweiten eindimensionalen Code aufweisen. Der zweite eindimensionale Code kann ein Code39, Code 128, Codebar, Interleaved 2of5, UPC, EAN, GS1, oder PDF417 sein. Der zweite eindimensionale Code ist jedoch nicht auf einen Code aus dieser Liste beschränkt. Auch mit dem zweiten eindimensionalen Code wird eine Möglichkeit bereitgestellt, weitere Informationen auf der Testvorrichtung anzuordnen. Mithilfe des zweiten eindimensionalen und/oder zweidimensionalen Codes in Kombination mit der Struktur, mit der der erste eindeutige Identifizierungscode kodiert ist, kann gewährleistet werden, dass die Testvorrichtung ein gewöhnliches Erscheinungsbild aufweist und bei einer unerlaubten Benutzung oder Vervielfältigung der Testvorrichtung die Struktur übersehen wird und somit der Kopierschutz nicht ohne Weiteres erkannt wird. Der zweite eindimensionale und/oder zweidimensionale Code kann beispielsweise so ausgebildet sein, dass er mit allen handelsüblichen Geräten/Scannern ausgelesen werden kann und beispielsweise durch seinen Dateninhalt auf eine Webseite verweist.

In einer Ausführungsform ist mit dem zweiten zweidimensionalen Code ein zweiter eindeutiger Identifizierungscode kodiert. Die im Zusammenhang mit dem ersten zweidimensionalen Code beschriebenen Merkmale, technischen Effekte und/oder Vorteile gelten zumindest in analoger Weise auch für den zweiten zweidimensionalen Code, sodass an dieser Stelle auf eine entsprechende Wiederholung verzichtet wird. Insbesondere wird dadurch, dass sowohl ein erster eindeutiger Identifizierungscode als auch ein zweiter eindeutiger Identifizierungscode verwendet werden, gewährleistet, dass eine doppelte Identifizierung der Testvorrichtung bereitgestellt wird, sodass selbst dann, wenn entweder der erste eindeutige Identifizierungscode oder der zweite eindeutige Identifizierungscode nicht ermittelt werden kann, zumindest der jeweils andere eindeutige Identifizierungscode ermittelt werden kann. Somit wird die Sicherheit von Testergebnissen weiter erhöht, insbesondere dann, wenn entweder die Struktur oder der zweite zweidimensionale Code auf der Testvorrichtung beschädigt, verunreinigt oder durch andere äußere Einflüsse nicht erfassbar ist. Weiter kann dadurch, dass sowohl ein erster eindeutiger Identifizierungscode als auch ein zweiter eindeutiger Identifizierungscode verwendet werden, gewährleistet, dass der erste eindeutige Identifizierungscode und der zweite eindeutige Identifizierungscode für unterschiedliche Zwecke verwendet werden können. Beispielsweise könnte der erste eindeutige Identifizierungscode für die Identifikation der Testvorrichtung in einer Behörde und der zweite eindeutige Identifizierungscode für die Identifikation der Testvorrichtung in einem Labor verwendet werden. Insbesondere können die unterschiedlichen eindeutigen Identifizierungscodes für unterschiedliche Zwecke, wie beispielsweise die Identifikation oder Registrierung während der Verwendung der Testvorrichtung durch eine sich testende Person, einen Arzt oder im Labor verwendet werden, wobei jedem Zweck ein bestimmter eindeutiger Identifizierungscode zugeordnet ist.

In einer Ausführungsform umschließt der zweite zweidimensionale Code das Ergebnisfeld. Wenn der zweite zweidimensionale Code das Ergebnisfeld umschließt wird gewährleistet, dass bei der optischen Erfassung der zweite zweidimensionale Code als Orientierungshilfe verwendet werden kann und bei der optischen Erfassung auf die Anordnung des Ergebnisfelds anhand der Anordnung des zweiten zweidimensionalen Codes rückgeschlossen werden kann. Zudem kann sichergestellt werden, dass bei einer optischen Erfassung des zweiten zweidimensionalen Codes, in jedem Fall das Ergebnisfeld miterfasst wird. Dadurch kann vermieden werden, dass der zweite zweidimensionale Code erfasst wird, beispielsweise abfotografiert wird, dabei versehentlich jedoch das Ergebnisfeld nicht miterfasst wird.

Erfindungsgemäß bildet die Struktur des Informationsfelds einen Abschnitt des zweiten zweidimensionalen Codes des Sicherheitsfelds. Wenn die Struktur des Informationsfelds einen Abschnitt des zweiten zweidimensionalen Codes des Sicherheitsfelds bildet, wird besonders platzsparend die Sicherheit von Testergebnissen erhöht. Gemäß einem zweiten Aspekt der Erfindung wird die eingangs genannte Aufgabe durch ein Testvorrichtungssystem mit den Merkmalen des Patentanspruchs 5 gelöst. Das Testvorrichtungssystem weist mehrere Testvorrichtungen nach dem ersten Aspekt der Erfindung auf.

Die eindeutigen Identifizierungscodes der Testvorrichtungen unterscheiden sich. Die im Zusammenhang mit der Testvorrichtung gemäß dem ersten Aspekt der Erfindung beschriebenen Merkmale, technischen Effekte und/oder Vorteile gelten zumindest in analoger Weise auch für das Testvorrichtungssystem gemäß dem zweiten Aspekt der Erfindung, sodass an dieser Stelle auf eine entsprechende Wiederholung verzichtet wird.

In einer Ausführungsform unterscheiden sich die ersten Erscheinungsformen der Testvorrichtungen des Testvorrichtungssystems. Wenn sich die ersten Erscheinungsformen der Testvorrichtungen unterscheiden, kann gewährleistet werden, dass ein Benutzer einer bestimmten Testvorrichtung nicht auf ein Testergebnis anhand der ersten Erscheinungsform rückschließen kann. Vielmehr ist es vorgesehen, dass beispielsweise mithilfe des ersten eindeutigen Identifizierungscodes ermittelt wird, auf welches konkrete Testergebnis anhand der konkret vorliegenden ersten Erscheinungsform rückgeschlossen werden kann. Dies kann beispielsweise durch einen Abgleich mit in einer Datenbank gespeicherter Zuordnungen von einer Vielzahl von ersten eindeutigen Identifizierungscodes und einer Vielzahl von ersten Erscheinungsformen und einer Vielzahl von zweiten Erscheinungsformen erfolgen, wobei jedem ersten eindeutigen Identifizierungscode eine erste Erscheinungsform und eine zweite Erscheinungsform zugeordnet sind. Insbesondere unterscheiden sich hierbei bei einem bestimmten ersten eindeutigen Identifizierungscode zugeordnete erste Erscheinungsform und zweite Erscheinungsform. Insbesondere dadurch, dass sich die ersten Erscheinungsformen der Testvorrichtungen des Testvorrichtungssystems voneinander unterscheiden kann beispielsweise vermieden werden, dass der Benutzer anhand der ersten Erscheinungsform versucht, die erste Erscheinungsform zu manipulieren, um aus der ersten Erscheinungsform gegebenenfalls die zweite Erscheinungsform zu erzeugen, um ein falsches Testergebnis zu erhalten. In der Datenbank können außerdem Personendaten eines Patienten, Datum und Uhrzeit einer Übertragung des eindeutigen Identifizierungscodes von der Auswerteeinheit an einen Server, und eine Geoposition der Erfassungseinheit zum Zeitpunkt der Erfassung des Ergebnisfelds und des Sicherheitsfelds gespeichert sein, die jeweils einem ersten eindeutigen Identifizierungscode zugeordnet sind.

Wie bereits beschrieben, kann die erste Erscheinungsform des Ergebnisfelds einen waagerechten Streifen in einem Kontrollfeld des Ergebnisfelds aufweisen. Dass sich die ersten Erscheinungsformen der Testvorrichtungen des Testvorrichtungssystems voneinander unterscheiden, kann beispielsweise dadurch erreicht werden, dass sich die Positionen der Kontrollfelder innerhalb der Ergebnisfelder der Testvorrichtungen voneinander unterscheiden. Beispielsweise kann somit von einem Benutzer vor dem Erscheinen eines waagerechten Streifens die Position des Kontrollfelds indem der waagerechte Streifen erscheint, nicht vorhergesagt werden. Somit kann der Benutzer nicht ohne Zweifel auf ein bestimmtes Testergebnis rückschließen. Insbesondere, wenn die Testvorrichtung zum Feststellen angepasst ist, ob die Flüssigkeit mehrere Inhaltsstoffe aufweist und jedem Inhaltsstoff ein entsprechender waagerechter Streifen in einem entsprechenden Kontrollfeld des Ergebnisfelds zugeordnet ist, ist es vorteilhaft, wenn sich die Positionen der Kontrollfelder für einen bestimmten Inhaltsstoff der Testvorrichtungen voneinander unterscheiden. Wenn bei einer derart ausgebildeten Testvorrichtung ein waagerechter Streifen an einer bestimmten Position erscheint, kann der Benutzer nicht auf den vorliegenden Inhaltsstoff rückschließen. Vielmehr ist vorgesehen, dass in einer Datenbank eine Zuordnungen von einer Vielzahl von ersten eindeutigen Identifizierungscodes und einer Vielzahl von ersten Erscheinungsformen und einer Vielzahl von zweiten Erscheinungsformen gespeichert ist. Eine Auswertung mithilfe der Datenbank kann anhand des ersten eindeutigen Identifizierungscodes darauf schließen, auf welches konkrete Testergebnis anhand der konkret vorliegenden ersten Erscheinungsform rückgeschlossen werden kann. Somit kann dann ein tatsächlich vorliegendes Testergebnis ermittelt werden. Das Testergebnis kann also nicht gefälscht werden, da der Benutzer anhand der Testvorrichtung nicht weiß, an welcher Position ein waagerechter Streifen für ein bestimmtes Testergebnis erscheint. Insbesondere für den Fall, dass, wie bereits beschrieben, sowohl Testvorrichtungen vorgesehen sind, bei denen die erste Erscheinungsform des Ergebnisfelds einen waagerechten Streifen in einem Kontrollfeld des Ergebnisfelds aufweist als auch Testvorrichtungen vorgesehen sind, bei denen die erste Erscheinungsform des Ergebnisfelds den waagerechten Streifen in dem Kontrollfeld des Ergebnisfelds nicht aufweist, kann ein Benutzer der Testvorrichtung nicht ohne Zweifel auf ein bestimmtes Testergebnis rückschließen.

Als dritter Aspekt, der nicht durch die Ansprüche gedeckt ist, wird ein Etikett beschrieben. Das Etikett weist ein Sicherheitsfeld auf. Das Sicherheitsfeld weist ein Informationsfeld mit einer Struktur auf. Mit der Struktur ist ein erster eindeutiger Identifizierungscode kodiert. Der Identifizierungscode kann nur aus der Struktur dekodiert werden, wenn die Struktur mit einer Mindestauflösung hergestellt ist. Das Etikett ist vorgesehen, an einer Testvorrichtung nach dem ersten Aspekt der Erfindung angebracht zu werden, sodass das Sicherheitsfeld des Etiketts das Sicherheitsfeld der Testvorrichtung bildet. Die im Zusammenhang mit der Testvorrichtung gemäß dem ersten Aspekt der Erfindung sowie die im Zusammenhang mit dem Testvorrichtungssystem gemäß dem zweiten Aspekt der Erfindung beschriebenen Merkmale, technischen Effekte und/oder Vorteile gelten zumindest in analoger Weise auch für das Etikett gemäß dem dritten Aspekt, sodass an dieser Stelle auf eine entsprechende Wiederholung verzichtet wird.

In einer Ausführungsform weist das Etikett eine mit einer Klebeschicht beschichtete Trägerschicht auf und das Sicherheitsfeld ist auf der Trägerschicht angeordnet. Mithilfe der Klebeschicht kann das Etikett an einer Testvorrichtung angebracht werden, sodass eine stoffschlüssige Verbindung zwischen dem Etikett und der Testvorrichtung hergestellt werden kann. Die Trägerschicht kann mechanisch verstärkt sein, und so beispielsweise die Steifigkeit der Trägerschicht erhöhen. Ein mechanische Verstärkung kann auch so gewählt sein, dass der Widerstand gegen Rissentstehung und/oder Rissausbreitung erhöht wird. Dies kann beispielsweise durch in die Trägerschicht eingebettete Fasern gewährleistet werden. Weiter kann das Etikett, insbesondere dann, wenn das Etikett die Klebeschicht oder eine Klebemasse, die nicht als Schicht ausgebildet sein muss, sondern beispielsweise eine Vielzahl nicht miteinander verbundener Klebemassenabschnitte aufweist, so gestaltet sein, dass das Etikett beim Ablösen von der Testvorrichtung beschädigt wird, sodass das Etikett nicht auf einer anderen Testvorrichtung wiederverwendet werden kann. Dies kann beispielsweise dadurch erreicht werden, dass das Etikett so ausgebildet ist, dass beim Ablösen des Etiketts von der Testvorrichtung, die Kraft, die zum Lösen der stoffschlüssigen Verbindung aufgebracht werden muss, größer ist als eine Maximalbelastungskraft des Etiketts, sodass das Etikett mechanisch versagt.

Als vierter Aspekt, der nicht von den Ansprüchen gedeckt ist, wird ein Etikettensystem beschrieben. Das Etikettensystem weist mehrere Etiketten nach dem dritten Aspekt auf. Die eindeutigen Identifizierungscodes der Etiketten unterscheiden sich. Die im Zusammenhang mit der Testvorrichtung gemäß dem ersten Aspekt, mit dem Testvorrichtungssystem gemäß dem zweiten Aspekt und mit dem Etikett gemäß dem dritten Aspekt beschriebenen Merkmale, technischen Effekte und/oder Vorteile gelten zumindest in analoger Weise auch für das Etikettensystem gemäß dem vierten Aspekt, sodass an dieser Stelle auf eine entsprechende Wiederholung verzichtet wird. Gemäß einem fünften Aspekt der Erfindung wird die eingangs genannte Aufgabe durch ein Verfahren mit den Merkmalen des Patentanspruchs 7 gelöst. Das Verfahren ist zum Feststellen, ob eine Flüssigkeit einen Inhaltsstoff aufweist angepasst. Das Verfahren weist folgende Schritte auf: gemeinsames, vorzugsweise gleichzeitiges optisches Erfassen des Ergebnisfelds und des Sicherheitsfelds einer Testvorrichtung nach dem ersten Aspekt der Erfindung durch eine Erfassungseinheit; Übertragen des optisch erfassten Ergebnisfelds und des optisch erfassten Sicherheitsfelds von der Erfassungseinheit an eine Auswerteeinheit; Rückschließen auf ein Testergebnis anhand des optisch erfassten Ergebnisfelds und auf den eindeutigen Identifizierungscode anhand des optisch erfassten Sicherheitsfelds durch die Auswerteeinheit; Zuordnen des Testergebnisses zu dem eindeutigen Identifizierungscode durch die Auswerteeinheit; Vergleichen des eindeutigen Identifizierungscodes mit gespeicherten eindeutigen Identifizierungscodes; Feststellen, ob die gespeicherten eindeutigen Identifizierungscodes den eindeutigen Identifizierungscode aufweisen; Feststellen, wenn die gespeicherten eindeutigen Identifizierungscodes den eindeutigen Identifizierungscode aufweisen, ob ein dem eindeutigen Identifizierungscode zugeordnetes Testergebnis gespeichert ist; und Speichern, wenn zu dem eindeutigen Identifizierungscode kein zugeordnetes Testergebnis gespeichert ist, des dem eindeutigen Identifizierungscode zugeordneten Testergebnisses. Dadurch, dass das dem eindeutigen Identifizierungscode zugeordnete Testergebnis nur dann gespeichert wird, wenn zu dem eindeutigen Identifizierungscode kein zugeordnetes Testergebnis gespeichert ist, wird eine Überprüfung vorgenommen, ob der erste eindeutige Identifizierungscode bereits verwendet wurde. Hierdurch kann gewährleistet werden, dass zu jedem eindeutigen Identifizierungscode nur ein Testergebnis gespeichert werden kann und dies nachträglich nicht überschrieben oder geändert wird. Die im Zusammenhang mit der Testvorrichtung gemäß dem ersten Aspekt, mit dem Testvorrichtungssystem gemäß dem zweiten Aspekt, mit dem Etikett gemäß dem dritten Aspekt und mit dem Etikettensystem gemäß dem vierten Aspekt beschriebenen Merkmale, technischen Effekte und/oder Vorteile gelten zumindest in analoger Weise auch für das Verfahren gemäß dem fünften Aspekt der Erfindung, sodass an dieser Stelle auf eine entsprechende Wiederholung verzichtet wird. Insbesondere ist vorgesehen, dass die Erfassungseinheit eine Erfassungseinheit eines Mobiltelefons ist, wie beispielsweise eine Kamera, insbesondere eine Digitalkamera, des Mobiltelefons. Vorzugsweise ist die Auswerteeinheit eine Auswerteeinheit eines Servers. Die Auswerteeinheit kann jedoch auch eine Auswerteeinheit des Mobiltelefons sein. Insbesondere für den Fall, dass die Auswerteeinheit eine Auswerteeinheit des Mobiltelefons ist, werden die folgenden Schritte bevorzugt serverseitig, insbesondere durch Server und Datenbank, durchgeführt: Vergleichen des eindeutigen Identifizierungscodes mit gespeicherten eindeutigen Identifizierungscodes; Feststellen, ob die gespeicherten eindeutigen Identifizierungscodes den eindeutigen Identifizierungscode aufweisen; Feststellen, wenn die gespeicherten eindeutigen Identifizierungscodes den eindeutigen Identifizierungscode aufweisen, ob ein dem eindeutigen Identifizierungscode zugeordnetes Testergebnis gespeichert ist; und Speichern, wenn zu dem eindeutigen Identifizierungscode kein zugeordnetes Testergebnis gespeichert ist, des dem eindeutigen Identifizierungscode zugeordneten Testergebnisses. Weiter werden dann, wenn die Auswerteeinheit eine Auswerteeinheit des Mobiltelefons ist, bevorzugt das Testergebnis und der eindeutigen Identifizierungscode von der Auswerteeinheit an den Server übertragen. In diesem Fall kann das Übermitteln großer Kamerabilder an den Server vermieden werden. Bevorzugt ist der Server mit einer Datenbank verbunden, um die einzelnen Schritte des Verfahrens durchzuführen.

In einer Ausführungsform weist das Verfahren außerdem folgenden Schritt auf: Ausgeben einer Bestätigungsnachricht, wenn das dem eindeutigen Identifizierungscode zugeordnete Testergebnis gespeichert wurde. Durch die Bestätigungsnachricht kann einem Benutzer der Testvorrichtung mittgeteilt werden, dass das Testergebnis gespeichert wurde. Da das dem eindeutigen Identifizierungscode zugeordnete Testergebnis lediglich dann gespeichert wird, wenn zu dem eindeutigen Identifizierungscode kein zugeordnetes Testergebnis gespeichert ist, kann darauf rückgeschlossen werden, dass mit der Testvorrichtung selbst oder mit einer Testvorrichtung, die mit einem Sicherheitsfeld mit einem Informationsfeld versehen ist, das eine Struktur aufweist, mit der ein identischer erster eindeutiger Identifizierungscode kodiert ist, kein vorheriger Test durchgeführt worden ist. Die Bestätigungsnachricht kann beispielsweise als Beweis, der auch als Zertifikat bezeichnet werden kann, verwendet werden und beispielsweise bei einem Grenzübertritt vorgezeigt werden, um eine mögliche Quarantäne zu verhindern, die für alle Personen vorgeschrieben sein könnte, die keine hohe Sicherheit ihres Testergebnisses oder sogar gar kein Testergebnis belegen können. Besonders bevorzugt kann die Bestätigungsnachricht von dem Mobiltelefon angezeigt werden.

In einer Ausführungsform weist das Verfahren außerdem folgende Schritte auf: Aufbringen einer Probe der Flüssigkeit auf das Probenfeld der Testvorrichtung; und Anbringen eines Etiketts nach dem dritten Aspekt an der Testvorrichtung, sodass das Sicherheitsfeld des Etiketts das Sicherheitsfeld der Testvorrichtung bildet. Zum einen kann vorgesehen sein, dass zunächst die Probe aufgebracht wird und anschließend das Etikett an der Testvorrichtung angebracht wird, sodass das Etikett während des Aufbringens der Probe noch nicht an der Testvorrichtung angebracht ist. Somit kann eine Manipulation des Etiketts während des Aufbringens der Probe vermieden werden. Alternativ ist es auch möglich, dass zuerst das Etikett an der Testvorrichtung angebracht wird und dann die Probe aufgebracht wird. Bevorzugt wird das Etikett bei der Produktion der Testvorrichtung an der Testvorrichtung angebracht. Besonders bevorzugt wird das Etikett nach der Produktion der Testvorrichtung und vor dem Aufbringen der Probe an der Testvorrichtung angebracht wird.

Auch wenn die Verfahrensschritte in einer bestimmten Reihenfolge beschrieben sind, ist die vorliegende Erfindung nicht auf diese Reihenfolge beschränkt. Vielmehr können die einzelnen Verfahrensschritte in beliebiger sinnvoller Reihenfolg, insbesondere auch zumindest abschnittsweise zeitlich parallel zueinander, durchgeführt werden.

Als sechster Aspekt, der nicht von den Ansprüchen gedeckt ist, wird ein System zur Datenverarbeitung beschrieben. System zur Datenverarbeitung umfasst Mittel zur Ausführung des Verfahrens nach dem fünften Aspekt der Erfindung. Die im Zusammenhang mit der Testvorrichtung gemäß dem ersten Aspekt, mit dem Testvorrichtungssystem gemäß dem zweiten Aspekt, mit dem Etikett gemäß dem dritten Aspekt, mit dem Etikettensystem gemäß dem vierten Aspekt und mit dem Verfahren gemäß dem fünften Aspekt der Erfindung beschriebenen Merkmale, technischen Effekte und/oder Vorteile gelten zumindest in analoger Weise auch für das System zur Datenverarbeitung gemäß dem sechsten Aspekt, sodass an dieser Stelle auf eine entsprechende Wiederholung verzichtet wird. In einer Ausführungsform umfassen die Mittel ein Mobiltelefon. In einer Ausführungsform umfassen die Mittel eine Datenbank.

Als siebter Aspekt, der nicht von den Ansprüchen gedeckt ist, wird ein Computerprogramm beschrieben.

Das Computerprogramm umfasst Befehle, die bei der Ausführung des Programms durch einen Computer diesen veranlassen, die Schritte des Verfahrens nach dem fünften Aspekt der Erfindung auszuführen. Die im Zusammenhang mit den anderen Aspekten beschriebenen Merkmale, technischen Effekte und/oder Vorteile gelten zumindest in analoger Weise auch für das Computerprogramm gemäß dem siebten Aspekt, sodass an dieser Stelle auf eine entsprechende Wiederholung verzichtet wird. In einer Ausführungsform umfassen die Mittel ein Mobiltelefon. In einer Ausführungsform umfassen die Mittel eine Datenbank. In einer Ausführungsform, sind zumindest eine Teilmenge der Befehle durch Befehle einer App gebildet.

Als achter Aspekt, der nicht von den Ansprüchen gedeckt ist, wird ein computerlesbares Medium beschrieben.

Auf dem computerlesbaren Medium ist das Computerprogramm nach dem siebten Aspekt gespeichert. Die im Zusammenhang mit den anderen Aspekten beschriebenen Merkmale, technischen Effekte und/oder Vorteile gelten zumindest in analoger Weise auch für das Computerlesbares Medium gemäß dem achten Aspekt, sodass an dieser Stelle auf eine entsprechende Wiederholung verzichtet wird.

Weitere Merkmale, Vorteile und Anwendungsmöglichkeiten der vorliegenden Erfindung ergeben sich aus der nachfolgenden Beschreibung der Ausführungsbeispiele und den Figuren. Dabei bilden alle beschriebenen und/oder bildlich dargestellten Merkmale für sich und in beliebiger Kombination den Gegenstand der Erfindung auch unabhängig von ihrer Zusammensetzung in den einzelnen Ansprüchen oder deren Rückbezügen. In den Figuren stehen weiterhin gleiche Bezugszeichen für gleiche oder ähnliche Objekte.
- Figur 1: zeigt eine schematische Ansicht einer ersten Ausführungsform einer erfindungsgemäßen Testvorrichtung,
- Figur 2: zeigt eine schematische Ansicht einer zweiten Ausführungsform der erfindungsgemäßen Testvorrichtung,
- Figur 3: zeigt eine schematische Ansicht einer dritten Ausführungsform der erfindungsgemäßen Testvorrichtung,
- Figur 4: zeigt eine schematische Ansicht einer vierten Ausführungsform der erfindungsgemäßen Testvorrichtung,
- Figur 5: zeigt ebenfalls eine schematische Ansicht der vierten Ausführungsform der erfindungsgemäßen Testvorrichtung,
- Figur 6: zeigt eine schematische Ansicht einer ersten Ausführungsform des erfindungsgemäßen Testvorrichtungssystems,
- Figur 7: zeigt eine schematische Ansicht einer zweiten Ausführungsform des erfindungsgemäßen Testvorrichtungssystems,
- Figur 8: zeigt eine schematische Ansicht einer Ausführungsform des Etiketts, und
- Figur 9: zeigt eine schematische Schnittansicht der in Figur 8 dargestellten Ausführungsform des Etiketts.

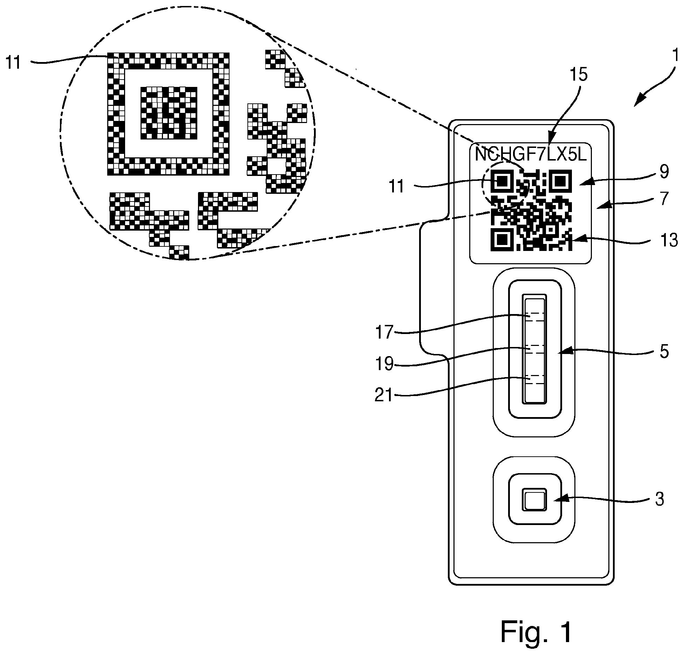

In Figur 1 ist eine schematische Ansicht einer ersten Ausführungsform einer erfindungsgemäßen Testvorrichtung 1 dargestellt. Die Testvorrichtung 1 weist ein Probenfeld 3, ein Ergebnisfeld 5 und ein an das Ergebnisfeld 5 angrenzendes Sicherheitsfeld 7 auf. Das Sicherheitsfeld 7 weist ein Informationsfeld 9 auf, das in dieser Ausführungsform durch einen QR-Code gebildet ist. In dem Informationsfeld 9 ist eine Struktur 11 ausgebildet, mit der ein erster eindeutiger Identifizierungscode kodiert ist, der in dieser Ausführungsform ein erster zweidimensionaler Code ist. Der erste zweidimensionale Code ist durch eine zweidimensionale Funktion repräsentiert. Die Struktur 11 ist erfindungsgemäß eine zweidimensionale Darstellung einer binarisierten Fourier-Transformierten dieser zweidimensionalen Funktion und in den dunklen Bereichen des QR-Codes in einer wie in der vergrößerten Ansicht dargestellten und noch näher beschriebenen Weise ausgebildet. Das Sicherheitsfeld 7 weist weiterhin einen zweiten zweidimensionalen Code 13 auf. Dieser zweite zweidimensionale Code 13 ist in dieser Ausführungsform durch den QR-Code als Ganzes einschließlich der dunklen und der hellen Bereiche gebildet. Der QR-Code weist die hellen und dunklen Bereiche auf, aufgrund deren Größe und Anordnung mit dem zweiten zweidimensionalen Code 13 ein zweiter eindeutiger Identifizierungscode kodiert ist.

Um die Struktur 11 zu bilden, durch die der erste eindeutige Identifizierungscode in Form des ersten zweidimensionalen Codes kodiert ist, sind die dunklen Bereiche des QR-Codes nicht gleichmäßig ausgebildet, sondern weisen eine in der nicht vergrößerten Ansicht in Figur 1 nicht erkennbare Strukturierung auf, die in der vergrößerten Ansicht schematisch dargestellt ist. Diese Strukturierung ist in dieser Ausführungsform durch weiße und schwarze Abschnitte innerhalb der dunklen Bereiche des QR-Codes gebildet. In dieser Ausführungsform sind die hellen Bereiche des QR-Codes weiß. Die Strukturierung kann jedoch auch in anderen Ausführungsformen durch Abschnitte mit unterschiedlichen Grauwerten oder farbig innerhalb der dunklen Bereiche des QR-Codes gebildet sein. Auch in dieser Ausführungsform sind die hellen Bereiche des QR-Codes weiß. In anderen Ausführungsformen kann die Strukturierung durch weiße und schwarze Abschnitte innerhalb der hellen Bereiche des QR-Codes gebildet sein. In diesem Fall sind die dunklen Bereiche des QR-Codes schwarz. Die Strukturierung kann auch in anderen Ausführungsformen durch Abschnitte mit unterschiedlichen Grauwerten oder farbig innerhalb der hellen Bereiche des QR-Codes gebildet sein. Auch in diesem Fall sind die dunklen Bereiche des QR-Codes schwarz.

Diese Strukturierung in den dunklen Bereichen des QR-Codes bildet die Struktur 11 im Sinne der vorliegenden Erfindung. Die Struktur 11 erstreckt sich damit über die dunklen Bereiche des zweiten zweidimensionalen Codes 13 in Form des QR-Codes. Die Struktur 11 des Informationsfelds 9 bildet somit einen Abschnitt des zweiten zweidimensionalen Codes 13. Außerdem ist die Struktur 11 innerhalb der dunklen Bereiche derart ausgebildet, dass sie mit einer Auflösung von 300 dpi hergestellt ist, d.h. es sind 300 schwarze oder weiße Abschnitte pro Zoll innerhalb der dunklen Bereiche des QR-Codes ausgebildet. Damit ist die Struktur 11 so ausgebildet, dass zum Dekodieren eine beispielsweise optische Erfassung mit einer Mindestauflösung von 300 dpi notwendig ist. Wenn umgekehrt die Struktur 11 mit einer geringeren Auflösung als 300 dpi hergestellt ist, kann der erste eindeutige Identifizierungscode nicht aus der Struktur 11 dekodiert werden, auch wenn er mit der richtigen Mindestauflösung erfasst wird. Ferner ist es auch denkbar, dass die Auflösung, mit der die Struktur 11 in die dunklen Bereiche eingebracht ist, höher ist, also beispielsweise 600 dpi oder größer als 600 dpi, wie 800 dpi, oder größer als 800 dpi.

Weiter weist das Sicherheitsfeld 7 einen dritten eindeutigen Identifizierungscode 15 auf. Der dritte eindeutige Identifizierungscode 15 in der in Figur 1 dargestellten Ausführungsform besteht aus der Zeichenfolge "NCHGF7LX5L". Das Sicherheitsfeld 7 weist somit den ersten eindeutigen Identifizierungscode in kodierter Form sowie den zweiten eindeutigen Identifizierungscode in kodierter Form, die zusammen in dem Informationsfeld 9 gebildet sind, und den dritten eindeutigen Identifizierungscode 15 in nicht-kodierter Form auf. Insbesondere kann in diesem Ausführungsbeispiel und in anderen Ausführungsbeispielen vorgesehen sein, dass der zweite eindeutige Identifizierungscode und der dritte eindeutige Identifizierungscode identisch sind. Hierdurch wird gewährleistet, dass für den Fall, dass der zweite eindeutige Identifizierungscode beispielsweise aufgrund einer Beschädigung oder aufgrund einer Verschmutzung nicht aus dem zweiten zweidimensionalen Code 13 dekodiert werden kann, kann der dritte eindeutige Identifizierungscode manuell beispielsweise durch Eintippen erfasst werden.

Da das Sicherheitsfeld 7 hier an das Ergebnisfeld 5 angrenzt, sind bei dieser Ausführungsform das Ergebnisfeld 5 und das Sicherheitsfeld 7 derart angeordnet, dass das Ergebnisfeld 5 und das Sicherheitsfeld 7 gemeinsam, vorzugsweise gleichzeitig, von einer Erfassungseinheit optisch erfasst werden können.

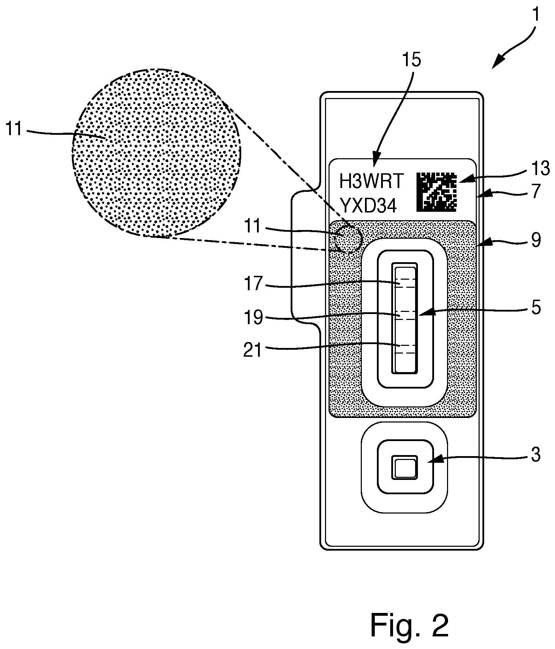

In Figur 2 ist eine schematische Ansicht einer zweiten Ausführungsform der erfindungsgemäßen Testvorrichtung 1 dargestellt. Die Testvorrichtung 1 weist ein Probenfeld 3, ein Ergebnisfeld 5 und ein an das Ergebnisfeld 5 angrenzendes und dieses umschließendes Sicherheitsfeld 7 auf. Das Sicherheitsfeld 7 weist ein das Ergebnisfeld 5 umschließendes Informationsfeld 9 mit einer Struktur 11 auf, die in der nicht vergrößerten Ansicht in Figur 2 nicht dargestellte weiße oder schwarze Abschnitte aufweist, die in der vergrößerten Ansicht schematisch dargestellt sind. Die Struktur 11 ist in dieser Ausführungsform durch weiße und schwarze Abschnitte gebildet. Die Struktur 11 kann jedoch auch in anderen Ausführungsformen durch Abschnitte mit unterschiedlichen Grauwerten oder farbig gebildet sein. Mit der Struktur 11 ist ein erster eindeutiger Identifizierungscode kodiert, der ein erster zweidimensionaler Code ist. Der erste zweidimensionale Code ist durch eine zweidimensionale Funktion repräsentiert. Die Struktur 11 ist erfindungsgemäß eine zweidimensionale Darstellung einer binarisierten Fourier-Transformierten dieser zweidimensionalen Funktion.

Außerhalb der Erfindung sind aber auch andere Möglichkeiten denkbar, den ersten zweidimensionalen Code zu kodieren, aber die Erfindung ist auf die Verwendung einer binarisierten Fourier-Transformierten beschränkt.

Auch bei dieser Ausführungsform ist die Struktur 11 so ausgebildet, dass zum Dekodieren eine beispielsweise optische Erfassung mit einer Mindestauflösung von 300 dpi notwendig ist, die Struktur also mit einer Auflösung von 300 dpi hergestellt ist. Wenn umgekehrt die Struktur 11 mit einer geringeren Auflösung als 300 dpi hergestellt ist, kann der erste eindeutige Identifizierungscode nicht aus der Struktur 11 dekodiert werden, auch wenn er mit der richtigen Mindestauflösung erfasst wird. Es ist ferner auch denkbar, dass die Auflösung, mit der die Struktur 11 in die dunklen Bereiche eingebracht ist, höher ist.

Das Sicherheitsfeld 7 der zweiten Ausführungsform weist einen zweiten zweidimensionalen Code 13 auf. Der zweite zweidimensionale Code 13 ist ein DataMatrix Code, wodurch eine besonders platzsparende Ausgestaltung der Testvorrichtung 1 bereitgestellt wird. Mit dem zweiten zweidimensionalen Code 13 ist ein zweiter eindeutiger Identifizierungscode kodiert. Weiter weist das Sicherheitsfeld 7 einen dritten eindeutigen Identifizierungscode 15 auf. Der dritte eindeutige Identifizierungscode 15 in der in Figur 2 dargestellten Ausführungsform besteht aus der Zeichenfolge "H3WRTYXD34". Das Sicherheitsfeld 7 weist somit den ersten eindeutigen Identifizierungscode in kodierter Form, den zweiten eindeutigen Identifizierungscode in kodierter Form und den dritten eindeutigen Identifizierungscode 15 in nicht-kodierter Form auf. Der erste eindeutige Identifizierungscode kann zur Überprüfung der Echtheit der Testvorrichtung 1 verwendet werden. Der zweite eindeutige Identifizierungscode kann nach einer automatischen Erfassung des zweiten zweidimensionalen Codes 13 in der Weiterverarbeitung verwendet werden. Der dritte eindeutige Identifizierungscode 15 ist in dieser Ausführungsform ein menschenlesbarer Code und kann für eine manuelle Erfassung verwendet werden.

Der den DataMatrix Code bildende zweite zweidimensionale Code 13 ist eine zweidimensionale Darstellung mit weißen Bereichen und schwarzen Bereichen. Die schwarzen Bereiche des zweiten zweidimensionalen Codes 13 weisen die Struktur 11 nicht auf. Das Informationsfeld 9 mit der Struktur 11 ist hier von dem zweiten zweidimensionalen Code 13 beabstandet angeordnet.

Da bei dieser Ausführungsform das Sicherheitsfeld 7 bzw. das Informationsfeld 9 das Ergebnisfeld 5 umgibt, sind bei dieser Ausführungsform das Ergebnisfeld 5 und das Sicherheitsfeld 7 derart angeordnet, dass das Ergebnisfeld 5 und das Sicherheitsfeld 7 gemeinsam, vorzugsweise gleichzeitig, von einer Erfassungseinheit optisch erfasst werden können.

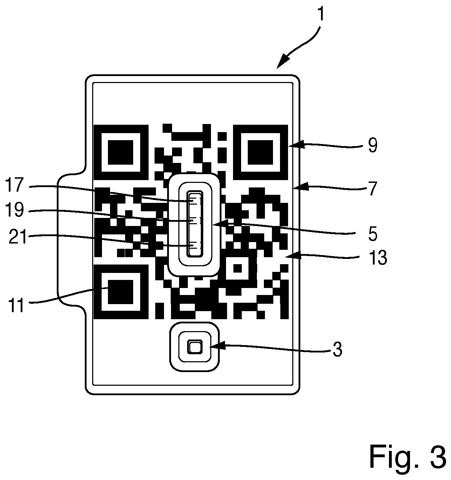

In Figur 3 ist eine schematische Ansicht einer dritten Ausführungsform der erfindungsgemäßen Testvorrichtung 1 dargestellt. Die Testvorrichtung 1 weist ein Probenfeld 3, ein Ergebnisfeld 5 und ein an das Ergebnisfeld 5 angrenzendes und dieses umschließendes Sicherheitsfeld 7 auf. Das Sicherheitsfeld 7 weist ein das Ergebnisfeld 5 umschließendes Informationsfeld 9 auf, das in dieser Ausführungsform auch durch einen QR-Code gebildet ist. In den dunklen Bereichen des QR-Codes ist eine Struktur 11 in der Weise ausgebildet, dass diese dunklen Bereiche in in Figur 3 nicht dargestellter Weise aus weißen und schwarzen Abschnitten aufgebaut sind. Die Struktur 11 ist auch hier so ausgebildet, dass zum Dekodieren eine beispielsweise optische Erfassung mit einer Mindestauflösung von 300 dpi notwendig ist, die Struktur also mit einer Auflösung von 300 dpi hergestellt ist. Wenn umgekehrt die Struktur 11 mit einer geringeren Auflösung als 300 dpi hergestellt ist, kann der erste eindeutige Identifizierungscode nicht aus der Struktur 11 dekodiert werden, auch wenn er mit der richtigen Mindestauflösung erfasst wird. Es ist auch denkbar, dass die Auflösung, mit der die Struktur 11 in die dunklen Bereiche eingebracht ist, höher ist.

Mit dieser Struktur 11 ist ein erster eindeutiger Identifizierungscode kodiert, der auch in dieser Ausführungsform ein erster zweidimensionaler Code ist. Der erste zweidimensionale Code ist durch eine zweidimensionale Funktion repräsentiert.

Die Struktur 11 ist erfindungsgemäß eine zweidimensionale Darstellung einer binarisierten Fourier-Transformierten dieser zweidimensionalen Funktion.

Das Sicherheitsfeld 7 weist außerdem einen das Ergebnisfeld 5 umschließenden zweiten zweidimensionalen Code 13 auf, der durch den QR-Code selbst gebildet ist. Mit diesem zweiten zweidimensionalen Code 13 ist ein zweiter eindeutiger Identifizierungscode kodiert. Das Sicherheitsfeld 7 weist somit den ersten eindeutigen Identifizierungscode in kodierter Form und den zweiten eindeutigen Identifizierungscode in kodierter Form auf.

Der den QR-Code bildende zweite zweidimensionale Code 13 ist eine zweidimensionale Darstellung mit weißen Bereichen und dunklen Bereichen. Die dunklen Bereiche des zweiten zweidimensionalen Codes 13 weisen die Struktur 11 auf. Die Struktur 11 erstreckt sich über die dunklen Bereiche des zweiten zweidimensionalen Codes 13. Die Struktur 11 des Informationsfelds 9 bildet somit einen Abschnitt des zweiten zweidimensionalen Codes 13. In dieser Ausführungsform weisen die dunklen Bereiche des zweiten zweidimensionalen Codes 13 die Struktur 11 auf. In anderen Ausführungsformen könne jedoch auch die weißen Bereiche des zweiten zweidimensionalen Codes 13 die Struktur 11 aufweisen, wobei die weißen Bereich in diesem Fall als helle Bereiche bezeichnet werden können. Da schließlich bei dieser Ausführungsform das Sicherheitsfeld 7 das Ergebnisfeld 5 umgibt, sind bei dieser Ausführungsform das Ergebnisfeld 5 und das Sicherheitsfeld 7 auch wieder so angeordnet, dass das Ergebnisfeld 5 und das Sicherheitsfeld 7 gemeinsam, vorzugsweise gleichzeitig, von einer Erfassungseinheit optisch erfasst werden können.

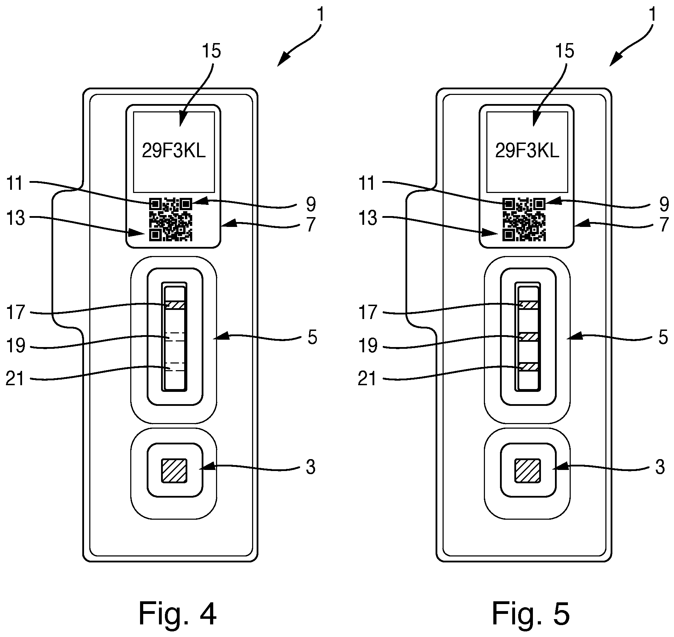

In Figur 4 und Figur 5 ist jeweils eine schematische Ansicht einer vierten Ausführungsform der erfindungsgemäßen Testvorrichtung 1 dargestellt. Die vierte Ausführungsform entspricht im Wesentlichen der ersten Ausführungsform, wobei sich der erste eindeutige Identifizierungscode, der zweite eindeutige Identifizierungscode und der dritte eindeutige Identifizierungscode 15, der aus der Zeichenfolge "29F3KL" besteht, der vierten Ausführungsform von dem ersten eindeutigen Identifizierungscode, dem zweiten eindeutigen Identifizierungscode und dem dritten eindeutige Identifizierungscode 15 der ersten Ausführungsform unterscheiden.

Die vierte Ausführungsform der erfindungsgemäßen Testvorrichtung 1 weist ein Probenfeld 3, ein Ergebnisfeld 5 und ein an das Ergebnisfeld 5 angrenzendes Sicherheitsfeld 7 auf. Das Sicherheitsfeld 7 weist weiter ein Informationsfeld 9 auf, das auch in dieser Ausführungsform durch einen QR-Code gebildet ist. In den dunklen Bereichen des QR-Codes ist eine Struktur 11 derart ausgebildet, dass die dunklen Bereiche in in Figur 4 und 5 nicht dargestellter Weise aus schwarzen und weißen Abschnitten aufgebaut sind. Die Struktur 11 ist auch hier so ausgebildet, dass zum Dekodieren eine beispielsweise optische Erfassung mit einer Mindestauflösung von 300 dpi notwendig ist, die Struktur also mit einer Auflösung von 300 dpi hergestellt ist. Wenn umgekehrt die Struktur 11 mit einer geringeren Auflösung als 300 dpi hergestellt ist, kann der erste eindeutige Identifizierungscode nicht aus der Struktur 11 dekodiert werden, auch wenn er mit der richtigen Mindestauflösung erfasst wird. Es ist auch möglich, dass die Auflösung, mit der die Struktur 11 in die dunklen Bereiche eingebracht ist, höher ist. Mit dieser Struktur 11 ist ein erster eindeutiger Identifizierungscode kodiert, der auch in dieser Ausführungsform ein erster zweidimensionaler Code ist. Der erste zweidimensionale Code ist durch eine zweidimensionale Funktion repräsentiert. Die Struktur 11 ist erfindungsgemäß eine zweidimensionale Darstellung einer binarisierten Fourier-Transformierten dieser zweidimensionalen Funktion.

Das Sicherheitsfeld 7 weist außerdem einen zweiten zweidimensionalen Code 13 auf, der durch den QR-Code selbst gebildet ist. Mit diesem zweiten zweidimensionalen Code 13 ist ein zweiter eindeutiger Identifizierungscode kodiert. Das Sicherheitsfeld 7 weist somit den ersten eindeutigen Identifizierungscode in kodierter Form und den zweiten eindeutigen Identifizierungscode in kodierter Form auf.

Zusätzlich weist das Sicherheitsfeld 7 der vierten Ausführungsform einer erfindungsgemäßen Testvorrichtung einen dritten eindeutigen Identifizierungscode 15, der aus der Zeichenfolge "29F3KL" besteht, auf.

Bei dieser Ausführungsform grenzt das Sicherheitsfeld 7 an das Ergebnisfeld 5 an, sodass auch bei dieser Ausführungsform das Ergebnisfeld 5 und das Sicherheitsfeld 7 derart angeordnet sind, dass das Ergebnisfeld 5 und das Sicherheitsfeld 7 gemeinsam, vorzugsweise gleichzeitig, von einer Erfassungseinheit optisch erfasst werden können.

Im Folgenden wird anhand der vierten Ausführungsform die Ausgestaltung der ersten, zweiten, dritten und vierten Ausführungsform beschrieben, die es erlaubt, mit der erfindungsgemäßen Testvorrichtung 1 festzustellen, ob eine Flüssigkeit einen Inhaltsstoff aufweist.

Auf das Probenfeld 3 kann eine Probe einer Flüssigkeit, wie beispielsweise Blut, aufgebracht werden. In den Figuren 4 und 5 ist das Probenfeld 3 schwarz dargestellt, wodurch eine auf das Probenfeld 3 aufgebrachte Flüssigkeit symbolisiert ist. Die Probe der Flüssigkeit kann einen Inhaltsstoff, wie beispielsweise Immunglobulin G oder Immunglobulin M, in mindestens einer Mindestmenge enthalten. Alternativ kann die Probe den Inhaltsstoff nicht in mindestens der Mindestmenge enthalten, beispielsweise dann, wenn die Probe den Inhaltsstoff nicht enthält. Das Ergebnisfeld 5 ist ausgestaltet, eine erste Erscheinungsform anzunehmen, wenn die Probe den Inhaltsstoff in mindestens der Mindestmenge enthält, und eine zweite Erscheinungsform anzunehmen, wenn die Probe den Inhaltsstoff nicht in mindestens der Mindestmenge enthält.

Wenn die Probe der Flüssigkeit auf das Probenfeld 3 aufgebracht wurde, erscheint wie in Figur 4 und 5 dargestellt sowohl dann, wenn die Probe den Inhaltsstoff in mindestens der Mindestmenge enthält, als auch dann, wenn die Probe den Inhaltsstoff nicht in mindestens der Mindestmenge enthält, in einem ersten Kontrollfeld 17 des Ergebnisfelds 5 ein waagerechter Streifen, sodass bei Erscheinen des waagerechten Streifens in dem ersten Kontrollfeld 17 auf die Funktionsfähigkeit der Testvorrichtung 1 rückgeschlossen werden kann. Sowohl die erste Erscheinungsform als auch die zweite Erscheinungsform des Ergebnisfeldes weist somit den waagerechten Streifen in dem ersten Kontrollfeld 17 auf. In einer anderen Ausführungsform kann der waagerechte Streifen in dem ersten Kontrollfeld 17 des Ergebnisfelds 5 auch dargestellt sein bevor die Probe der Flüssigkeit auf das Probenfeld 3 aufgebracht wurde. Dieser waagerechte Streifen in dem ersten Kontrollfeld 17 des Ergebnisfelds 5 ist somit immer sichtbar und gewährleistet, dass ein Nutzer der Testvorrichtung erkennt wie ein waagerechter Streifen aussieht, wenn er erscheint, so dass der Nutzer klar vergleichen kann, ob ein Strich sichtbar wurde oder nicht. Der waagerechte Streifen in dem ersten Kontrollfeld 17 wird häufig mit "C" gekennzeichnet und kann auch als "Control" bezeichnet werden.

Weiter erscheint dann, wenn die Probe der Flüssigkeit auf das Probenfeld 3 aufgebracht wurde und die Probe den Inhaltsstoff Immunglobulin G (IgG) in mindestens der Mindestmenge enthält, in einem zweiten Kontrollfeld 19 des Ergebnisfelds 5 ein waagerechter Streifen, sodass bei Erscheinen des waagerechten Streifens in dem zweiten Kontrollfeld 19 auf das Vorhandensein von Immunglobulin G in mindestens der Mindestmenge rückgeschlossen werden kann. Dann, wenn die Probe der Flüssigkeit auf das Probenfeld 3 aufgebracht wurde und die Probe den Inhaltsstoff Immunglobulin G nicht in mindestens der Mindestmenge enthält, erscheint in dem zweiten Kontrollfeld 19 des Ergebnisfelds 5 kein waagerechter Streifen, sodass bei Nichterscheinen des waagerechten Streifens in dem zweiten Kontrollfeld 19 auf das Nichtvorhandensein oder das Vorhandensein von Immunglobulin G nicht in mindestens der Mindestmenge rückgeschlossen werden kann. Bezogen auf Immunglobulin G weist die erste Erscheinungsform des Ergebnisfelds 5 den waagerechten Streifen in dem zweiten Kontrollfeld 19 auf. Bezogen auf Immunglobulin G weist die zweite Erscheinungsform des Ergebnisfelds 5 den waagerechten Streifen in dem zweiten Kontrollfeld 19 nicht auf. Bezogen auf Immunglobulin G kann somit die Erscheinungsform des Ergebnisfelds 5 in Figur 4 als zweite Erscheinungsform bezeichnet werden und die Erscheinungsform des Ergebnisfelds 5 in Figur 5 kann als erste Erscheinungsform bezeichnet werden.

Außerdem erscheint dann, wenn die Probe der Flüssigkeit auf das Probenfeld 3 aufgebracht wurde und die Probe den Inhaltsstoff Immunglobulin M (IgM) in mindestens der Mindestmenge enthält, in einem dritten Kontrollfeld 21 des Ergebnisfelds 5 ein waagerechter Streifen, sodass bei Erscheinen des waagerechten Streifens in dem dritten Kontrollfeld 21 auf das Vorhandensein von Immunglobulin M in mindestens der Mindestmenge rückgeschlossen werden kann. Dann, wenn die Probe der Flüssigkeit auf das Probenfeld 3 aufgebracht wurde und die Probe den Inhaltsstoff Immunglobulin M nicht in mindestens der Mindestmenge enthält, erscheint in dem dritten Kontrollfeld 21 des Ergebnisfelds 5 kein waagerechter Streifen, sodass bei Nichterscheinen des waagerechten Streifens in dem dritten Kontrollfeld 21 auf das Nichtvorhandensein oder das Vorhandensein von Immunglobulin M nicht in mindestens der Mindestmenge rückgeschlossen werden kann. Bezogen auf Immunglobulin M weist die erste Erscheinungsform des Ergebnisfelds 5 den waagerechten Streifen in dem dritten Kontrollfeld 21 auf. Bezogen auf Immunglobulin M weist die zweite Erscheinungsform des Ergebnisfelds 5 den waagerechten Streifen in dem dritten Kontrollfeld 21 nicht auf. Bezogen auf Immunglobulin M kann somit die Erscheinungsform des Ergebnisfelds 5 in Figur 4 als zweite Erscheinungsform bezeichnet werden und die Erscheinungsform des Ergebnisfelds 5 in Figur 5 kann als erste Erscheinungsform bezeichnet werden.

Mit der Testvorrichtung 1 kann somit festgestellt werden, ob eine Flüssigkeit, wie beispielsweise Blut, einen Inhaltsstoff, wie beispielsweise Immunglobulin G oder Immunglobulin M aufweist. Insbesondere kann mit der Testvorrichtung 1 festgestellt werden, ob die Flüssigkeit zwei Inhaltsstoffe, nämlich Immunglobulin G und Immunglobulin M aufweist. Bezogen diese beiden Inhaltstoffe kann die Erscheinungsform des Ergebnisfelds 5 in Figur 4 als zweite Erscheinungsform bezeichnet werden und die Erscheinungsform des Ergebnisfelds 5 in Figur 5 kann als erste Erscheinungsform bezeichnet werden.

Für die erste, zweite, dritte und vierte Ausführungsform gilt, wie bereits beschrieben, dass mit der Struktur 11 des Informationsfelds 9 der erste eindeutige Identifizierungscode kodiert ist. Der erste eindeutige Identifizierungscode kann aus der Struktur 11 nur dekodiert werden, wenn die Struktur 11 mit einer Mindestauflösung hergestellt ist. Wenn die Struktur 11 mit einer Auflösung hergestellt ist, die geringer als die Mindestauflösung ist, kann der eindeutige Identifizierungscode nicht aus der Struktur 11 dekodiert werden, da in diesem Fall die für die Dekodierung notwendigen Informationen nicht in der Struktur 11 enthalten sind.

Das Ergebnisfeld 5 und das Sicherheitsfeld 7 sind weiterhin wie erläutert derart angeordnet, dass das Ergebnisfeld 5 und das Sicherheitsfeld 7 gemeinsam, vorzugsweise gleichzeitig, von einer Erfassungseinheit optisch erfasst werden können.

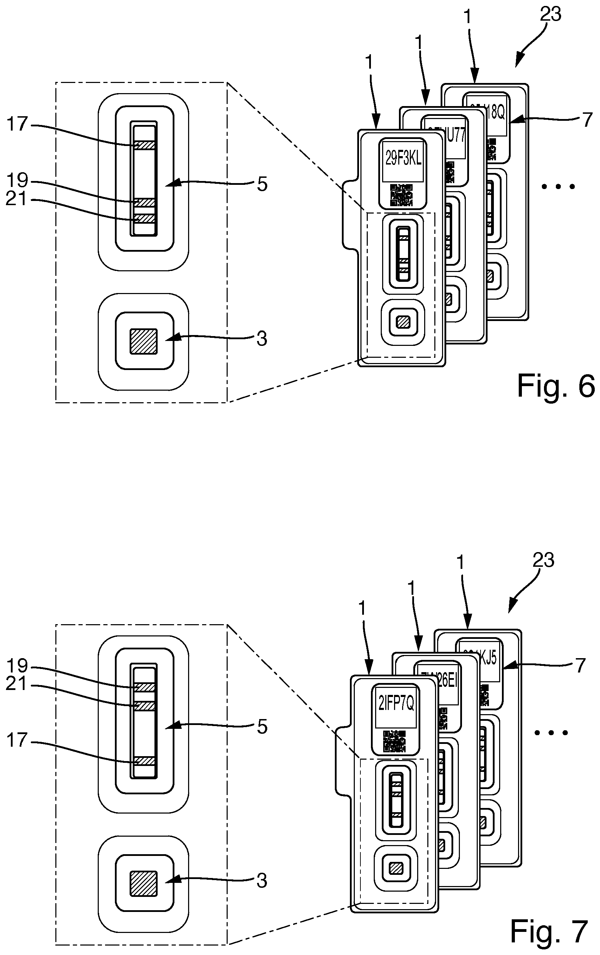

In Figur 6 ist eine schematische Ansicht einer ersten Ausführungsform des erfindungsgemäßen Testvorrichtungssystems 23 dargestellt. Das Testvorrichtungssystem 23 weist mehrere Testvorrichtungen 1 gemäß der ersten, zweiten, dritten oder vierten Ausführungsform auf. Die ersten eindeutigen Identifizierungscodes der Testvorrichtungen 1 unterscheiden sich. Außerdem unterscheiden sich die zweiten eindeutigen Identifizierungscodes der Testvorrichtungen 1. Schließlich unterscheiden sich auch die dritten eindeutigen Identifizierungscodes 15 der Testvorrichtungen 1. Bezogen auf die beiden Inhaltstoffe Immunglobulin G und Immunglobulin M kann die Erscheinungsform des Ergebnisfelds 5 in Figur 6 als erste Erscheinungsform bezeichnet werden. Die ersten Erscheinungsformen der Testvorrichtungen 1 der ersten Ausführungsform des Testvorrichtungssystems 23 sind identisch. Die Testvorrichtungen 1 der ersten Ausführungsform des Testvorrichtungssystems 23 sind beispielsweise in einer ersten Charge gefertigt. Insbesondere ist die Anordnung des ersten, zweiten und dritten Kontrollfeldes 17, 19, 21 innerhalb der ersten Charge des Testvorrichtungssystems 23 gleich. Denkbar ist jedoch auch, dass sich die ersten Erscheinungsformen der Testvorrichtungen 1 unterscheiden.

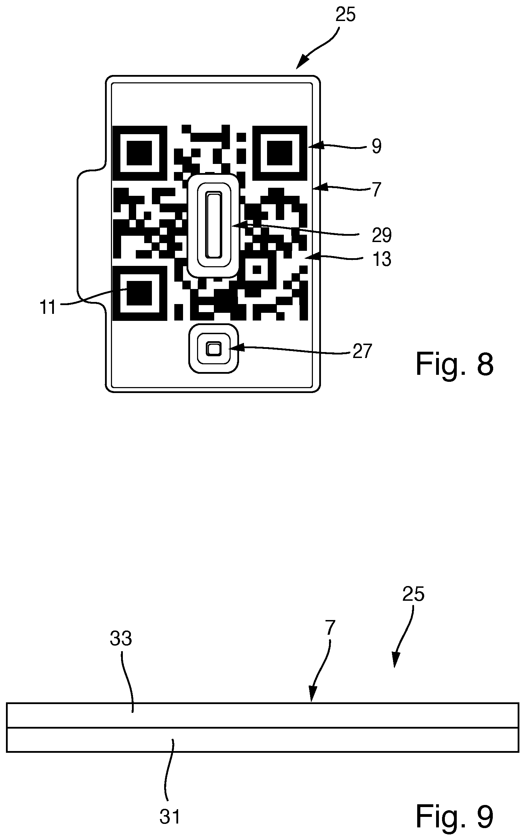

In Figur 7 ist eine schematische Ansicht einer zweiten Ausführungsform des erfindungsgemäßen Testvorrichtungssystems 23 dargestellt. Das Testvorrichtungssystem 23 weist mehrere Testvorrichtungen 1 gemäß der ersten, zweiten, dritten oder vierten Ausführungsform auf. Die ersten eindeutigen Identifizierungscodes der Testvorrichtungen 1 unterscheiden sich. Außerdem unterscheiden sich die zweiten eindeutigen Identifizierungscodes der Testvorrichtungen 1. Schließlich unterscheiden sich auch die dritten eindeutigen Identifizierungscodes 15 der Testvorrichtungen 1. Bezogen auf die beiden Inhaltstoffe Immunglobulin G und Immunglobulin M kann die Erscheinungsform des Ergebnisfelds 5 in Figur 7 als erste Erscheinungsform bezeichnet werden. Die ersten Erscheinungsformen der Testvorrichtungen 1 der zweiten Ausführungsform des Testvorrichtungssystems 23 sind identisch. Die Testvorrichtungen 1 der zweiten Ausführungsform des Testvorrichtungssystems 23 sind beispielsweise in einer zweiten Charge gefertigt. Insbesondere ist die Anordnung des ersten, zweiten und dritten Kontrollfeldes 17, 19, 21 innerhalb der zweiten Charge des Testvorrichtungssystems gleich. Denkbar ist jedoch auch, dass sich die ersten Erscheinungsformen der Testvorrichtungen 1 unterscheiden. Beispielsweise kann das Testvorrichtungssystem 23 sowohl Testvorrichtungen 1 mit der ersten Erscheinungsform gemäß Figur 6 als auch Testvorrichtungen 1 mit der ersten Erscheinungsform gemäß Figur 7 aufweisen, sodass ein Benutzer der Testvorrichtung 1 vor dem Aufbringen der Probe der Flüssigkeit auf das Probenfeld 3 nicht voraussehen kann, wie die Streifen der ersten Erscheinungsform 1 des Ergebnisfelds 5 in dem Ergebnisfeld 5 angeordnet sind. Die Testvorrichtungen 1 können in einer Vielzahl von Chargen gefertigt werden, wobei sich die Anordnung des ersten, zweiten und dritten Kontrollfeldes 17, 19, 21 der Chargen voneinander unterscheiden, sodass für einen Benutzer einer bestimmten Testvorrichtung 1 nicht ersichtlich ist, welches Testergebnis tatsächlich vorliegt. Vielmehr ist der Benutzer darauf angewiesen, dass anhand einer Zuordnung des ersten eindeutigen Identifizierungscodes und eines ersten Erscheinungsbilds auf ein bestimmtes Testergebnis rückgeschlossen wird. In Figur 8 ist eine schematische Ansicht einer Ausführungsform des Etiketts 25 dargestellt. Das Etikett 25 ist vorgesehen, an einer Testvorrichtung 1 gemäß der dritten Ausführungsform angebracht zu werden, sodass das Sicherheitsfeld 7 des Etiketts 25 das Sicherheitsfeld 7 der Testvorrichtung 1 bildet.

Das Etikett 25 weist ein Sicherheitsfeld 7 auf, das wiederum ein Informationsfeld 9 umfasst. Das Informationsfeld 9 ist in dieser Ausführungsform auch durch einen QR-Code gebildet ist. In den dunklen Bereichen des QR-Codes ist eine Struktur 11 in der Weise ausgebildet, dass diese dunklen Bereiche in Figur 8 nicht dargestellter Weise aus schwarzen und weißen Abschnitten aufgebaut sind. Die Struktur 11 ist auch hier so ausgebildet, dass zum Dekodieren eine beispielsweise optische Erfassung mit einer Mindestauflösung von 300 dpi notwendig ist, die Struktur also mit einer Auflösung von 300 dpi hergestellt ist. Wenn die Struktur 11 mit einer geringeren Auflösung als 300 dpi hergestellt ist, kann der erste eindeutige Identifizierungscode nicht aus der Struktur 11 dekodiert werden. Es ist auch möglich, dass die Auflösung, mit der die Struktur 11 in die dunklen Bereiche eingebracht ist, höher ist. Mit dieser Struktur 11 ist ein erster eindeutiger Identifizierungscode kodiert, der auch in dieser Ausführungsform ein erster zweidimensionaler Code ist. Der erste zweidimensionale Code kann durch eine zweidimensionale Funktion repräsentiert sein. Die Struktur 11 ist auch bei dieser Ausführungsform eine zweidimensionale Darstellung einer binarisierten Fourier-Transformierten dieser zweidimensionalen Funktion. Das Sicherheitsfeld 7 weist außerdem einen zweiten zweidimensionalen Code 13 auf, der durch den QR-Code selbst gebildet ist. Mit diesem zweiten zweidimensionalen Code 13 ist ein zweiter eindeutiger Identifizierungscode kodiert. Das Sicherheitsfeld 7 weist somit den ersten eindeutigen Identifizierungscode in kodierter Form und den zweiten eindeutigen Identifizierungscode in kodierter Form auf.

Der den QR-Code bildende zweite zweidimensionale Code 13 ist eine zweidimensionale Darstellung mit weißen Bereichen und dunklen Bereichen. Die dunklen Bereiche des zweiten zweidimensionalen Codes 13 weisen die Struktur 11 auf. Die Struktur 11 erstreckt sich über die dunklen Bereiche des zweiten zweidimensionalen Codes 13. Die Struktur 11 des Informationsfelds 9 bildet somit einen Abschnitt des zweiten zweidimensionalen Codes 13.

Das Etikett 25 weist eine erste Ausnehmung 27 auf, die gewährleistet, dass dann, wenn das Etikett 25 an der Testvorrichtung 1 angebracht ist, das Probenfeld 3 der Testvorrichtung 1 (siehe Figur 3) so freiliegt, dass eine Probe der Flüssigkeit auf das Probenfeld 3 aufgebracht werden kann. Weiter weist das Etikett 25 eine zweite Ausnehmung 29 auf, die gewährleistet, dass dann, wenn das Etikett 25 an der Testvorrichtung 1 angebracht ist, das Ergebnisfeld 5 der Testvorrichtung 1 (siehe Figur 3) so freiliegt, dass das Ergebnisfeld 5, insbesondere das erste Kontrollfeld 17, das zweite Kontrollfeld 19 und das dritte Kontrollfeld 21, so freiliegen, dass das Ergebnisfeld 5, insbesondere das erste Kontrollfeld 17, das zweite Kontrollfeld 19 und das dritte Kontrollfeld 21, von einer Erfassungseinheit optisch erfasst werden können.

In Figur 9 ist eine schematische Schnittansicht der in Figur 8 dargestellten Ausführungsform des Etiketts 25 dargestellt. Das Etikett 25 weist eine mit einer Klebeschicht 31 beschichtete Trägerschicht 33 auf. Das Sicherheitsfeld 7 ist auf der Trägerschicht 33 angeordnet. Mithilfe der Klebeschicht 31 kann das Etikett 25 an einer Testvorrichtung 1 angebracht werden, sodass eine stoffschlüssige Verbindung zwischen dem Etikett 25 und der Testvorrichtung 1 hergestellt werden kann. Die Trägerschicht 33 und die Klebeschicht 31 sind in dieser Ausführungsform derart gestaltet, dass sich das Etikett 25 beim Entfernen zerstört. Dies kann beispielsweise dadurch erreicht werden, dass eine auf das Substrat angepasster Kleber oder/und eine Sicherheitsanstanzung oder mehrere Sicherheitsanstanzungen in dem Etikett vorgesehen sind.

Mehrere Etiketten 25 der Ausführungsform des Etiketts 25 können zusammen ein Etikettensystem bilden. In dem Etikettensystem unterscheiden sich die ersten eindeutigen Identifizierungscodes der Etiketten 25. Ebenso unterscheiden sich die zweiten eindeutigen Identifizierungscodes der Etiketten 25.

Wie bereits beschrieben, kann mithilfe der Testvorrichtung 1 gemäß der ersten, zweiten, dritten und vierten Ausführungsform festgestellt werden, ob eine Flüssigkeit einen Inhaltsstoff aufweist. Die Testvorrichtung 1 kann somit in einem Ausführungsbeispiel eines Verfahrens zum Feststellen, ob eine Flüssigkeit einen Inhaltsstoff aufweist, verwendet werden. Dieses Ausführungsbeispiel eines Verfahrens umfasst die folgenden Schritte: Anbringen eines Etiketts 25 an der Testvorrichtung 1, sodass das Sicherheitsfeld 7 des Etiketts 25 das Sicherheitsfeld 7 der Testvorrichtung 1 bildet; Aufbringen einer Probe der Flüssigkeit auf das Probenfeld 3 der Testvorrichtung 1; gemeinsames, vorzugsweise gleichzeitiges, optisches Erfassen des Ergebnisfelds 5 und des Sicherheitsfelds 7 der Testvorrichtung 1 durch eine Erfassungseinheit; Übertragen des optisch erfassten Ergebnisfelds 5 und des optisch erfassten Sicherheitsfelds 7 von der Erfassungseinheit an eine Auswerteeinheit; Rückschließen auf ein Testergebnis anhand des optisch erfassten Ergebnisfelds 5 und auf den ersten eindeutigen Identifizierungscode anhand des optisch erfassten Sicherheitsfelds 7 durch die Auswerteeinheit; Zuordnen des Testergebnisses zu dem ersten eindeutigen Identifizierungscode durch die Auswerteeinheit; Vergleichen des ersten eindeutigen Identifizierungscodes mit gespeicherten eindeutigen Identifizierungscodes; Feststellen, ob die gespeicherten ersten eindeutigen Identifizierungscodes den eindeutigen Identifizierungscode aufweisen; Feststellen, wenn die gespeicherten ersten eindeutigen Identifizierungscodes den ersten eindeutigen Identifizierungscode aufweisen, ob ein dem ersten eindeutigen Identifizierungscode zugeordnetes Testergebnis gespeichert ist; Speichern, wenn zu dem ersten eindeutigen Identifizierungscode kein zugeordnetes Testergebnis gespeichert ist, des dem ersten eindeutigen Identifizierungscode zugeordneten Testergebnisses; und Ausgeben einer Bestätigungsnachricht, wenn das dem ersten eindeutigen Identifizierungscode zugeordnete Testergebnis gespeichert wurde.

Das hier beschriebene Verfahren wird von Mitteln eines Systems zur Datenverarbeitung ausgeführt. Weiter ist ein Computerprogramm vorgesehen, das Befehle umfasst, die bei der Ausführung des Programms durch einen Computer diesen veranlassen, die Schritte des Verfahrens auszuführen. Außerdem ist ein computerlesbares Medium vorgesehen, auf dem das Computerprogramm gespeichert ist.

Ergänzend sei darauf hingewiesen, dass "aufweisend" keine anderen Elemente oder Schritte ausschließt und "ein" oder "eine" keine Vielzahl ausschließt. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können. Bezugszeichen in den Ansprüchen sind nicht als Einschränkung anzusehen.

### Bezugszeichen

- 1: Testvorrichtung
- 3: Probenfeld
- 5: Ergebnisfeld
- 7: Sicherheitsfeld
- 9: Informationsfeld
- 11: Struktur
- 13: zweiter zweidimensionaler Code
- 15: dritter eindeutiger Identifizierungscode
- 17: erstes Kontrollfeld
- 19: zweites Kontrollfeld
- 21: drittes Kontrollfeld
- 23: Testvorrichtungssystem
- 25: Etikett
- 27: erste Ausnehmung
- 29: zweite Ausnehmung
- 31: Klebeschicht
- 33: Trägerschicht

## Patentansprüche

1. Testvorrichtung (1) zum Feststellen, ob eine Flüssigkeit einen Inhaltsstoff aufweist,
mit einem Probenfeld (3), auf das eine Probe der Flüssigkeit aufgebracht werden kann,
mit einem Ergebnisfeld (5), das ausgestaltet ist, eine erste Erscheinungsform anzunehmen, wenn die Probe den Inhaltsstoff in mindestens einer Mindestmenge enthält, und eine zweite Erscheinungsform anzunehmen, wenn die Probe den Inhaltsstoff nicht in mindestens der Mindestmenge enthält, und
mit einem Sicherheitsfeld (7) mit einem Informationsfeld (9) mit einer Struktur (11), mit der ein erster eindeutiger Identifizierungscode kodiert ist, wobei der erste eindeutige Identifizierungscode aus der Struktur (11) nur dekodiert werden kann, wenn diese mit einer Mindestauflösung hergestellt ist,
wobei das Ergebnisfeld (5) und das Sicherheitsfeld (7) derart angeordnet sind, dass das Ergebnisfeld (5) und das Sicherheitsfeld (7) gemeinsam, vorzugsweise gleichzeitig, von einer Erfassungseinheit optisch erfasst werden können, wobei der erste eindeutige Identifizierungscode ein erster zweidimensionaler Code ist, **dadurch gekennzeichnet, dass** die Struktur (11) des Informationsfelds (9) eine zweidimensionale Darstellung einer binarisierten Fourier-Transformierten einer den ersten zweidimensionalen Code repräsentierenden zweidimensionalen Funktion ist und wobei das Sicherheitsfeld (7) einen zweiten zweidimensionalen Code (13) aufweist und wobei die Struktur (11) des Informationsfelds (9) einen Abschnitt des zweiten zweidimensionalen Codes (13) des Sicherheitsfelds (7) bildet.

2. Testvorrichtung (1) nach Anspruch 1, wobei das Ergebnisfeld (5) und das Sicherheitsfeld (7) voneinander beabstandet sind oder
wobei das Ergebnisfeld (5) und das Sicherheitsfeld (7) aneinander angrenzen.

3. Testvorrichtung (1) nach einem der vorhergehenden Ansprüche, wobei das Sicherheitsfeld (7) das Ergebnisfeld (5) umschließt, und/oder
wobei das Informationsfeld (9) das Ergebnisfeld (5) umschließt.

4. Testvorrichtung (1) nach einem der vorhergehenden Ansprüche,
wobei mit dem zweiten zweidimensionalen Code (13) ein zweiter eindeutiger Identifizierungscode kodiert ist, und/oder
wobei der zweite zweidimensionale Code (13) das Ergebnisfeld (5) umschließt.

5. Testvorrichtungssystem (23) mit mehreren Testvorrichtungen (1) nach einem der vorhergehenden Ansprüche, wobei sich die ersten eindeutigen Identifizierungscodes der Testvorrichtungen (1) unterscheiden.

6. Testvorrichtungssystem (23) nach Anspruch 5, wobei sich die ersten Erscheinungsformen der Testvorrichtungen (1) unterscheiden.

7. Verfahren zum Feststellen, ob eine Flüssigkeit einen Inhaltsstoff aufweist, wobei das Verfahren folgende Schritte aufweist:
gemeinsames, vorzugsweise gleichzeitiges, optisches Erfassen des Ergebnisfelds (5) und des Sicherheitsfelds (7) einer Testvorrichtung (1) nach einem der Ansprüche 1 bis 6 durch eine Erfassungseinheit,
Übertragen des optisch erfassten Ergebnisfelds (5) und des optisch erfassten Sicherheitsfelds (7) von der Erfassungseinheit an eine Auswerteeinheit,
Rückschließen auf ein Testergebnis anhand des optisch erfassten Ergebnisfelds (5) und auf den ersten eindeutigen Identifizierungscode anhand des optisch erfassten Sicherheitsfelds (7) durch die Auswerteeinheit,
Zuordnen des Testergebnisses zu dem ersten eindeutigen Identifizierungscode durch die Auswerteeinheit,
Vergleichen des ersten eindeutigen Identifizierungscodes mit gespeicherten eindeutigen Identifizierungscodes,
Feststellen, ob die gespeicherten ersten eindeutigen Identifizierungscodes den eindeutigen Identifizierungscode aufweisen,
Feststellen, wenn die gespeicherten ersten eindeutigen Identifizierungscodes den ersten eindeutigen Identifizierungscode aufweisen, ob ein dem ersten eindeutigen Identifizierungscode zugeordnetes Testergebnis gespeichert ist, und
Speichern, wenn zu dem ersten eindeutigen Identifizierungscode kein zugeordnetes Testergebnis gespeichert ist, des dem ersten eindeutigen Identifizierungscode zugeordneten Testergebnisses.

8. Verfahren nach Anspruch 7, wobei das Verfahren außerdem folgenden Schritt aufweist:
Ausgeben einer Bestätigungsnachricht, wenn das dem ersten eindeutigen Identifizierungscode zugeordnete Testergebnis gespeichert wurde und/oder
wobei das Verfahren außerdem folgende Schritte aufweist:
Aufbringen einer Probe der Flüssigkeit auf das Probenfeld (3) der Testvorrichtung (1), und
Anbringen eines Etiketts (25) nach einem der Ansprüche 7 oder 8 an der Testvorrichtung (1), sodass das Sicherheitsfeld (7) des Etiketts (25) das Sicherheitsfeld (7) der Testvorrichtung (1) bildet.

## Claims

1. Test device (1) for determining whether a liquid has a substance,
having a sample field (3) to which a sample of the liquid can be applied,
having a result field (5) which is configured to take on a first appearance when the sample contains the substance in at least a minimum quantity, and to take on a second appearance when the sample does not contain the substance in at least the minimum quantity, and
having a security field (7) having an information field (9) having a structure (11), with which a first unique identification code is encoded, wherein the first unique identification code can only be decoded from the structure (11) when this is produced with a minimum resolution,
wherein the result field (5) and the security field (7) are arranged in such a way that joint, preferably simultaneous, optical detection of the result field (5) and the security field (7) by a detection unit can occur, wherein the first unique identification code is a first two-dimensional code, **characterised in that** the structure (11) of the information field (9) is a two-dimensional depiction of a binary coded Fourier transform of a two-dimensional function representing the first two-dimensional code, and wherein the security field (7) has a second two-dimensional code (13) and wherein the structure (11) of the information field (9) forms a section of the second two-dimensional code (13) of the security field (7).

2. Test device (1) according to claim 1, wherein the result field (5) and the security field (7) are spaced apart from each other or wherein the result field (5) and the security field (7) border each other.

3. Test device (1) according to one of the preceding claims, wherein the security field (7) encloses the result field (5), and/or wherein the information field (9) encloses the result field (5).

4. Test device (1) according to one of the preceding claims,
wherein a second unique identification code is encoded with the second two-dimensional code (13), and/or
wherein the second two-dimensional code (13) encloses the result field (5).

5. Test device system (23) having several test devices (1) according to one of the preceding claims, wherein the first unique identification codes of the test devices (1) differ.

6. Test device system (23) according to claim 5, wherein the first appearances of the test devices (1) differ.

7. Method for determining whether a liquid has a substance, wherein the method comprises the following steps:
jointly, preferably simultaneously, optically detecting the result field (5) and the security field (7) of a test device (1) according to one of claims 1 to 6 by a detection unit,
transmitting the optically detected result field (5) and the optically detected security field (7) from the detection unit to an evaluation unit,
deducing, by the evaluation unit, a test result using the optically detected result field (5) and the first unique identification code using the optically detected security field (7), assigning the test result to the first unique identification code by the evaluation unit,
comparing the first unique identification code with a stored unique identification code,
determining whether the stored first unique identification codes have the unique identification code,
determining whether a test result assigned to the first unique identification code is stored when the stored first unique identification codes have the first unique identification code, and
storing the test result assigned to the first unique identification code when no test result assigned to the first unique identification code is stored.

8. Method according to claim 7, wherein the method further comprises the following step:
output of an actuation message, when the test result assigned to the first unique identification codes has been stored,
and/or
wherein the method further comprises the following steps:
applying a sample of the liquid to the sample field (3) of the test device (1), and
applying a label (25) according to one of claims 7 or 8 to the test device (1) so that the security field (7) of the label (25) forms the security field (7) of the test device (1).

## Revendications

1. Dispositif de test (1) pour déterminer si un liquide contient un ingrédient,
avec un champ d'échantillon (3) sur lequel un échantillon du liquide peut être appliqué,
avec un champ de résultat (5) qui est configuré pour prendre un premier aspect si l'échantillon contient l'ingrédient en au moins une quantité minimale et pour prendre un second aspect si l'échantillon ne contient pas l'ingrédient en au moins la quantité minimale, et
avec un champ de sécurité (7) avec un champ d'information (9) avec une structure (11), avec laquelle est codé un premier code d'identification unique, dans lequel le premier code d'identification unique ne peut être décodé à partir de la structure (11) que si celle-ci est réalisée avec une résolution minimale,
dans lequel le champ de résultat (5) et le champ de sécurité (7) sont agencés de manière à ce que le champ de résultat (5) et le champ de sécurité (7) puissent être détectés optiquement ensemble, de préférence simultanément, par une unité de détection, dans lequel le premier code d'identification unique est un premier code bidimensionnel, **caractérisé en ce que** la structure (11) du champ d'information (9) est une représentation bidimensionnelle d'une transformée de Fourier binaire d'une fonction bidimensionnelle représentant le premier code bidimensionnel et dans lequel le champ de sécurité (7) présente un second code bidimensionnel (13) et dans lequel la structure (11) du champ d'information (9) forme une section du second code bidimensionnel (13) du champ de sécurité (7).

2. Dispositif de test (1) selon la revendication 1, dans lequel le champ de résultat (5) et le champ de sécurité (7) sont espacés l'un de l'autre ou dans lequel le champ de résultat (5) et le champ de sécurité (7) sont adjacents l'un à l'autre.

3. Dispositif de test (1) selon l'une quelconque des revendications précédentes, dans lequel le champ de sécurité (7) entoure le champ de résultat (5), et/ou dans lequel le champ d'information (9) entoure le champ de résultat (5).

4. Dispositif de test (1) selon l'une quelconque des revendications précédentes,
dans lequel un second code d'identification unique est codé avec le second code bidimensionnel (13), et/ou
dans lequel le second code bidimensionnel (13) entoure le champ de résultat (5).

5. Système de dispositifs de test (23) avec plusieurs dispositifs de test (1) selon l'une quelconque des revendications précédentes, dans lequel les premiers codes d'identification uniques des dispositifs de test (1) diffèrent.

6. Système de dispositifs de test (23) selon la revendication 5, dans lequel les premiers aspects des dispositifs de test (1) diffèrent.

7. Procédé pour déterminer si un liquide présente un ingrédient, dans lequel le procédé présente les étapes suivantes :
la détection optique commune, de préférence simultanée, du champ de résultat (5) et du champ de sécurité (7) d'un dispositif de test (1) selon l'une quelconque des revendications 1 à 6 par une unité de détection,
la transmission du champ de résultat (5) détecté optiquement et du champ de sécurité (7) détecté optiquement de l'unité de détection à une unité d'évaluation,
la déduction d'un résultat de test à l'aide du champ de résultat (5) détecté optiquement et du premier code d'identification unique à l'aide du champ de sécurité (7) détecté optiquement par l'unité d'évaluation,
l'attribution du résultat de test au premier code d'identification unique par l'unité d'évaluation,
la comparaison du premier code d'identification unique à des codes d'identification uniques enregistrés,
le fait de déterminer si les premiers codes d'identification uniques enregistrés présentent le code d'identification unique,
le fait de déterminer, lorsque les premiers codes d'identification uniques enregistrés présentent le premier code d'identification unique, si un résultat de test attribué au premier code d'identification unique est enregistré, et
l'enregistrement, lorsqu'aucun résultat de test attribué au premier code d'identification unique n'est enregistré, du résultat de test attribué au premier code d'identification unique.

8. Procédé selon la revendication 7, dans lequel le procédé présente en outre l'étape suivante :
l'émission d'un message de confirmation lorsque le résultat de test attribué au premier code d'identification unique a été enregistré et/ou
dans lequel le procédé présente en outre les étapes suivantes :
l'application d'un échantillon du liquide sur le champ d'échantillon (3) du dispositif de test (1), et
la fixation d'une étiquette (25) selon l'une quelconque des revendications 7 ou 8 sur le dispositif de test (1), de sorte que le champ de sécurité (7) de l'étiquette (25) forme le champ de sécurité (7) du dispositif de test (1).
